# EUROPEAN PATENT SPECIFICATION

(11) **EP 3 402 533 B1**
(45) Date of publication and mention of the grant of the patent: **04.08.2021**
(21) Application number: 17738930.1
(22) Date of filing: 12.01.2017
(51) Int. Cl.: A61K 48/00, A61P 3/00, C12N 5/10, C12N 9/40, C12N 15/09

(54) **METHODS AND COMPOSITIONS FOR THE TREATMENT OF NEUROLOGIC DISEASE**
VERFAHREN UND ZUSAMMENSETZUNGEN ZUR BEHANDLUNG VON NEUROLOGISCHER ERKRANKUNG
MÉTHODES ET COMPOSITIONS POUR LE TRAITEMENT D'UNE MALADIE NEUROLOGIQUE

(30) Priority: 15.01.2016 US 201662279394 P; 26.02.2016 US 201662300271 P; 28.04.2016 US 201662328925 P
(43) Date of publication of application: 21.11.2018
(73) Proprietor: Sangamo Therapeutics, Inc., Brisbane, CA 94005 (US); Regents of the University of Minnesota, Minneapolis, MN 55455-2020 (US)
(72) Inventor: DEKELVER, Russell, Brisbane California 94005 (US); MCIVOR, R. Scott, Minneapolis Minnesota 55455-2020 (US); OU, Li, Minneapolis Minnesota 55455-2020 (US); WECHSLER, Thomas, Brisbane California 94005 (US); WHITLEY, Chester B., Minneapolis Minnesota 55455-2020 (US); LAOHARAWEE, Kanut, Minneapolis Minnesota 55455-2020 (US)
(74) Representative: Marshall, Cameron John
(86) International application number: PCT/US2017/013189
(87) International publication number: WO 2017/123757

(56) References cited:
- WO-A1-2014/186579
- WO-A2-2009/018122
- WO-A2-2012/054723
- US-A1- 2010 183 577
- US-A1- 2014 017 212
- US-A1- 2015 159 172
- US-B2- 7 090 836
- M. CARDONE ET AL: "Correction of Hunter syndrome in the MPSII mouse model by AAV2/8-mediated gene delivery", HUMAN MOLECULAR GENETICS, vol. 15, no. 7, 1 January 2006 (2006-01-01), pages 1225-1236, XP055226439, gb ISSN: 0964-6906, DOI: 10.1093/hmg/ddl038
- VINICIA ASSUNTA POLITO ET AL: "IDS Crossing of the Blood-Brain Barrier Corrects CNS Defects in MPSII Mice", AMERICAN JOURNAL OF HUMAN GENETICS, AMERICAN SOCIETY OF HUMAN GENETICS, CHICAGO, IL, US, vol. 85, no. 2, 14 August 2009 (2009-08-14), pages 296-301, XP002692097, ISSN: 0002-9297, DOI: 10.1016/J.AJHG.2009.07.011
- OU ET AL.: 'High-dose enzyme replacement therapy in murine Hurler syndrome' MOL GENET METAB vol. 111, no. 2, 19 September 2013, pages 116 - 122, XP028821342

## Description

### TECHNICAL FIELD

The present disclosure is in the field of the treatment of neurologic diseases, and especially lysosomal diseases with central nervous system involvement, and including mucopolysaccharidosis type I (MPS I), and both the "attenuated forms" such as Scheie syndrome (MPS IS) and Hurler-Scheie syndrome (MPS H-S), as well as the more rapidly progressive form Hurler syndrome (MPS IH) and mucopolysaccharidosis type II (MPS II), also known as Hunter syndrome, and gene therapy.

### BACKGROUND

Gene therapy holds enormous potential for a new era of human therapeutics. These methodologies will allow treatment for conditions that heretofore have not been addressable by standard medical practice. One area that is especially promising is the ability to add a transgene to a cell to cause that cell to express a product that previously not being produced in that cell. Examples of uses of this technology include the insertion of a gene encoding a therapeutic protein, insertion of a coding sequence encoding a protein that is somehow lacking in the cell or in the individual and insertion of a sequence that encodes a structural nucleic acid such as a microRNA.

Transgenes can be delivered to a cell by a variety of ways, such that the transgene becomes integrated into the cell's own genome and is maintained there. In recent years, a strategy for transgene integration has been developed that uses cleavage with site-specific nucleases for targeted insertion into a chosen genomic locus (*see, e.g.,* co-owned U.S. Patent 7,888,121). Nucleases, such as zinc finger nucleases (ZFNs), transcription activator-like effector nucleases (TALENs), or nuclease systems such as the CRISPR/Cas system (utilizing an engineered guide RNA), are specific for targeted genes and can be utilized such that the transgene construct is inserted by either homology directed repair (HDR) or by end capture during non-homologous end joining (NHEJ) driven processes. *See, e.g.,* U.S. Patent Nos 9,394,531; 9,255,250; 9,200,266; 9,045,763; 9,005,973; 9,150,847; 8,956,828; 8,945,868; 8,703,489; 8,586,526; 6,534,261; 6,599,692; 6,503,717; 6,689,558; 7,067,317; 7,262,054; 7,888,121; 7,972,854; 7,914,796; 7,951,925; 8,110,379; 8,409,861; U.S. Patent Publications 20030232410; 20050208489; 20050026157; 20050064474; 20060063231; 20080159996; 201000218264; 20120017290; 20110265198;20130137104;20130122591;20130177983;20130196373; 20150056705 and 20150335708.

Targeted loci include "safe harbor" loci such as the AAVS1, HPRT, Albumin and CCR5 genes in human cells, and *Rosa*26 in murine cells. *See, e.g.,* U.S. Patent Nos. 9,394,545; 9,222,105; 9,150,847; 8,895,264; 8,771,985; 8,110,379; 7,951,925; U.S. Publication Nos. 20100218264; 20110265198; 20150056705 and 20150159172). Nuclease-mediated integration offers the prospect of improved transgene expression, increased safety and expressional durability, as compared to classic integration approaches that rely on random integration of the transgene, since it allows exact transgene positioning for a minimal risk of gene silencing or activation of nearby oncogenes, which in turn allows for the provision of proteins for the treatment and/or prevention of diseases.

While delivery of the transgene to the target cell is one hurdle that must be overcome to fully enact this technology, another issue that must be conquered is insuring that after the transgene is inserted into the cell and is expressed, the gene product so encoded must reach the necessary location with the organism, and be made in sufficient local concentrations to be efficacious. For diseases characterized by the lack of a protein or by the presence of an aberrant non-functional one, delivery of a transgene encoded wild type protein can be extremely helpful.

Lysosomal storage diseases (LSDs) are a group of rare metabolic monogenic diseases characterized by the lack of functional individual lysosomal proteins normally involved in the breakdown of waste lipids, mucopolysaccharides (i.e. glycosoaminoglycans (GAG)). These diseases are characterized by a buildup of these compounds in the cell since it is unable to process them for recycling due to the mis-functioning of a specific enzyme. The pathophysiology of LSD was initially thought to be tied to the simple deposition of GAG, but current research has led to an appreciation of the complexities of these diseases. GAG storage appears to lead to the perturbation of cellular, tissue and organ homeostasis, and has also been linked to increased secretion of cytokine and inflammatory modulators leading to an activation of the inflammatory response (Muenzer (2014) Mol Gen Metabol 111:63-72).

The most common examples are Gaucher disease (glucocerebrosidase deficiency- gene name: GBA), Fabry disease (α galactosidase A deficiency: GLA), Hunter syndrome (also called MPS II, iduronate 2-sulfatase deficiency: IDS), Hurler syndrome (also called MPS IH, alpha-L iduronidase deficiency: IDUA), Pompe disease (alpha-glucosidase: GAA) and Niemann-Pick disease type A and type B (sphingomyelin phosphodiesterase 1 deficiency- SMPD1) diseases. When grouped all together, LSDs have an incidence in the population of about 1 in 7000 births. Treatment options include enzyme replacement therapy (ERT) in which the missing enzyme is given to the patient, usually through intravenous injection in large doses. Such treatment is only to treat the symptoms and is not curative, thus the patient must be given repeated dosing of these proteins for the rest of their lives, and potentially may develop neutralizing antibodies to the injected protein.

MPS I is associated with IDUA (alpha-L iduronidase) deficiency and occurs approximately once in every 100,000 births. The IDUA enzyme deficiency results in the accumulation of GAG in lysosomes of several organs, including the brain, which leads to clinical manifestations of the disease. GAG can be detected in urine, plasma, and tissues, and can serve as biomarkers for disease progression. It is an autosomal recessive disorder, and heterozygote subjects with one normal IDUA gene and one mutant copy produce a reduced amount of the enzyme but will likely be asymptomatic because enough enzyme is produced from the wild type gene. For the patient, symptoms of Hurler syndrome most often appear between ages 3 and 8 and can include abnormal bones in the spine, claw hand, cloudy corneas, deafness, halted growth, heart valve problems, joint disease, including stiffness, an intellectual disability that gets worse over time, and thick, coarse facial features with low nasal bridge. Infants with even severe Hurler syndrome appear normal at birth and facial symptoms may become more noticeable during the first 2 years of life. As the children age, they develop a short stature (maximum of approximately four feet) and often die before age 10 from obstructive airway disease, respiratory infections, or cardiac complications.

Clinically, MPS I is divided into three categories: Hurler syndrome, Hurler-Scheie syndrome, and Scheie syndrome, so named after the doctors who originally described the condition. Hurler syndrome is generally regarded as the most severe and the brain is affected; Scheie syndrome is the most 'mild' [or attenuated]. Many people fall between the two and are referred to as Hurler-Scheie.

Hunter syndrome (Mucopolysaccharidosis Type II, MPS II) is a rare X-linked lysosomal disorder caused by lack of functional iduronate 2-sulfatase enzyme (IDS) and subsequent accumulation of glycosaminoglycans (GAGs) in affected individuals. Hunter syndrome affects approximately 1 in every 100,000 to 150,000 males born and can occur very rarely in females. MPS II is a variable, progressive, multisystem disorder where in most patients, symptoms are severe and death occurs at an early age (between 10- 20 years of age) although in patients with a more attenuated form of the disorder, survival into their 50s and 60s has been reported (Wraith et al (2008) Eur J Pediatr 167:267-277).

Clinically, patients on the severe end of the spectrum appear normal at birth but are often diagnosed between 18 and 36 months of age based on symptoms as recurrent respiratory infections, developmental delays, stiff joints and hip dysplasia, recurrent umbilical and inguinal hernias, and the development of the typical facial features associated with MPS II (prominent forehead, a nose with a flattened bridge, an enlarged tongue and an enlarged head). As the disease progresses, the clinical manifestations become multi-systemic, effecting the pulmonary and cardiac systems as well as the musculoskeletal system and the CNS. The differences between severe and attenuated forms are mainly due to the presence of neurodegeneration and mental impairment.

Standard treatment for MPS I and MPS II are either enzyme replacement therapy (ERT), varying from $100,000 to $500,000, or hematopoietic stem cell transplantation (HSCT), costing approximately $200,000 for a single round, or a combination of the two. These treatment options have substantive drawbacks however. For ERT, some studies of long term treatment have shown a statistically significant association between duration of ERT use and worsening quality of life (Aronovich and Hackett (2015) Mol Gen Metabol 114: 83-93). Additionally, ERT is used for amelioration of somatic disease (without CNS involvement), and is inefficient for treatment of neurologic disease because the exogenously given enzyme does not cross the blood brain barrier (Muenzer *ibid*)*.* For HSCT, there is a significant rate of mortality (at least 10%) due to the HSCT process (Aronovich and Hackett, *ibid*)*.* Thus, the most severe MPS I and MPS II patients with CNS impairment are underserved by the currently available treatments. US 2015/159172 describes methods and compositions for insertion of transgene sequences encoding proteins that are aberrantly expressed in diseases such as lysosomal storage diseases. Cardone et al. (Human Molecular Genetics (2006), 15(7): 1225-1236) discloses the administration of an adenovirus-associated virus (AAV) vector comprising an IDS transgene driven by a thyroxin-binding globulin (TBG) promoter in an MPSII mouse model. WO 2014/186579 relates to direct CNS delivery of a recombinant AAV vector encoding a gene product associated with a CNS disease. Polito et al. (American Journal of Human Genetics (2009), 85(2): 296-301) describes the use of an AAV2 vector containing a hIDS transgene under the control of the CMV promoter in studies relating to the correction of CNS defects in MPSII mice. US 2014/017212 describes nucleases and methods of using such nucleases for inserting a sequence encoding a therapeutic protein into a cell for the treatment and/or prevention of a lysosomal storage disease.

Thus, there remains a need for methods and compositions that can be used to treat MPS I and MPS II disease, including treatment through genome editing, for instance, to deliver a transgene encoding a gene product such that expression of the transgene will be at a therapeutically relevant level.

### SUMMARY

Disclosed herein are methods and compositions for treating and/or preventing Hurler (or MPS I) disease and for treating and/or preventing Hunter (MPS II) syndrome. The scope of the invention is defined by the appended claims.

The invention provides a promoterless donor vector and an expression vector for use in a method of reducing or preventing central nervous system (CNS) impairment in a subject with mucopolysaccharidosis type II (MPS II) or mucopolysaccharidosis type I (MPS I), wherein the promoterless donor vector comprises a transgene encoding human iduronate-2-sulfatase (hIDS) or human α-L-iduronidase (hIDUA) and the expression vector encodes a pair of zinc finger nucleases (ZFNs) targeted to an endogenous albumin gene, the method comprising administering to the subject the promoterless donor vector and expression vector, wherein the transgene is integrated into the albumin gene in a liver cell following cleavage of the albumin gene such that the encoded hIDUA or hIDS is expressed and secreted from the liver and further wherein the hIDUA or hIDS secreted from the liver is found in the central nervous system (CNS) (e.g., crosses the blood-brain barrier) of the subject such that the CNS impairment is reduced or prevented.

The transgene encodes a full length or truncated IDUA protein for MPS I or a full length or truncated IDS protein for MPS II that treats the disease - a protein that is lacking or deficient in MPS I or MPS II, which when supplied by the IDUA or IDS transgene treats and/or prevents MPS I or MPS II, respectively. The IDUA or IDS protein is excreted from the target (liver) cell such that it is able to affect or be taken up by other cells that do not harbor the transgene (bystander effect or cross correction). Creation of a population of altered cells in a patient will supply that needed protein activity to treat (*e.g.,* reduce or eliminate one or more symptoms) MPS I or MPS II disease.

The CNS impairment may comprise cognitive deficits (*e.g.,* loss of learning ability is decreased as compared to an untreated individual); the transgene(s) may modulate(s) levels of glycosoaminoglycans in the brain of the subject; and/or neuronal or glial vacuolation in the subject may be reduced or eliminated in the CNS (brain and/or spinal cord). The methods may further comprise administering an immunosuppressant to the subject prior to and after administration of the donor vector and the expression vector. The pair of ZFNs may comprise zinc finger proteins as shown in Table 1. The ZFN expression vectors and/or donor vectors may be adeno-associated vectors (AAV), which may be administered by any method, including via intravenous injection. In certain embodiments, the ZFN:ZFN:Donor ratio administered to the subject is 1:1:8.

In some embodiments, the active expressed IDUA and/or IDS expressed reaches 1, 2, 3, 4, 5, 10, 15, 20, 25, 30, 40, 50, 60, 70, 80, 90, 100, 200, 300 or greater percent of the endogenous levels in the subject with MPSI or MPS II disease. In some embodiments, the active IDUA or IDS enzyme expressed reaches 1, 2, 3, 4, 5, 10, 15, 20, 25, 30, 40, 50, 60, 70, 80, 90, 100, 200, 300 or greater percent of enzyme levels found in wild type individuals. The corrective IDUA or IDS transgene is inserted into subject in need thereof such that the replacement protein is produced *in vivo.* The expressed IDUA or IDS protein is excreted from the cell (*e.g.* via exportation into the blood) to act on or be taken up by other cells that lack the IDUA or IDS transgene (for example by receptor-mediated endocytosis via the mannose receptor or the mannose-6-phosphate receptor). Thus, cells expressing the IDUA or IDS transgene secrete the IDUA or IDS protein which then acts on one or more secondary tissues (e.g., brain, muscle, bones, heart, lung, spleen, etc.). The target tissue (which expresses the IDUA or IDS transgene) is the liver.

The corrective IDUA or IDS gene may comprise the wild type sequence of the functioning IDUA or IDS gene (including functional fragments of wild-type), while in other embodiments, the sequence of the corrective IDUA or IDS transgene is altered in some manner, for example to give enhanced biological activity (*e.g*., optimized codons to increase biological activity and/or truncation of the IDUA- or IDS-encoding sequence). In preferred embodiments, the compositions of the invention produce active IDUA or IDS enzyme in an MPSI or MPS II subject in need thereof. The expressed IDUA and/or IDS enzymes are able to degrade glycosoaminoglycans (GAGs). In some embodiments, GAGs are reduced in specific tissues of the body, including liver, spleen, kidney, lung, heart, muscle and brain. In preferred embodiments, the GAG levels in any tissue are reduced 1, 2, 3, 4, 5, 10, 15, 20, 25, 30, 40, 50, 60, 70, 80, 90 or 100% or any value there between as compared to an untreated MPSI or MPS II subject.

The IDUA or IDS transgene is inserted into the genome of a target cell using zinc-finger nucleases (ZFNs), which include a DNA-binding molecule that binds to a target site in a region of interest in the genome of the cell and one or more nuclease domains (*e.g*., cleavage domain and/or cleavage half-domain). Cleavage domains and cleavage half domains can be obtained, for example, from various restriction endonucleases, Cas proteins (class 1 or class 2) and/or homing endonucleases. The zinc finger domain recognizes a target site in an albumin gene. *See, e.g.,* U.S. Publication No. 2014001721,U.S. Patent Nos. 7,888,121; 7,972,854; 7,914,796; 7,951,925; 8,110,379; 8,409,861; 8,586,526; U.S. Patent Publications 20030232410; 20050208489; 20050026157; 20060063231; 20080159996; 201000218264; 20120017290; 20110265198; 20130137104; 20130122591; 20130177983; 20130177960 and 20140017212.

The nuclease (*i.e.* ZFN system) is supplied in polynucleotide form - an expression vector comprising a polynucleotide, encoding the pair of ZFNs targeted to an endogenous albumin gene, optionally operably linked to a promoter. In one embodiment, the expression vector is a viral vector.

The donor IDUA or IDS sequence is in a separate vector (*e.g.,* Ad, AAV or LV vector or mRNA). Such insertion of a donor nucleotide sequence into the target locus results in the expression of the IDUA or IDS transgene under control of the target locus's (albumin) endogenous genetic control elements. In some aspects, insertion of the IDUA or IDS transgene into a target gene (albumin), results in expression of an intact IDUA or IDS protein sequence and lacks any amino acids encoded by the target (albumin). In other aspects, the expressed exogenous IDUA or IDS protein is a fusion protein and comprises amino acids encoded by the IDUA or IDS transgene and by the endogenous locus into which the IDUA or IDS transgene is inserted (*e.g*., from the endogenous target locus or, alternatively from sequences on the transgene that encode sequences of the target locus). The target is an albumin gene. In some instances, the endogenous sequences will be present on the amino (N)-terminal portion of the exogenous IDUA protein, while in others, the endogenous sequences will be present on the carboxy (C)- terminal portion of the exogenous IDUA or IDS protein. In other instances, endogenous sequences will be present on both the N- and C-terminal portions of the IDUA exogenous protein. The endogenous sequences may include full-length wild-type or mutant endogenous sequences or, alternatively, may include partial endogenous amino acid sequences. The therapeutic IDUA or IDS protein product encoded on the transgene is exported out of the cell to affect or be taken up by cells lacking the transgene, *i.e.,* the cell is a liver cell which releases the therapeutic IDUA or IDS protein into the blood stream to act on distal tissues (*e.g*., brain, heart, muscle, etc.).

In one embodiment, the IDUA or IDS transgene is expressed from the albumin promoter following insertion into the albumin locus. The biologic encoded by the IDUA or IDS transgene then may be released into the blood stream if the transgene is inserted into a hepatocyte *in vivo.* In some aspects, the IDUA or IDS transgene is delivered to the liver *in vivo* in a viral vector through intravenous or other injection.

In further aspects, the vector comprising the transgene donor is delivered to a suitable target cell *in vivo,* such that the IDUA or IDS therapeutic protein encoded by the transgene donor is released into the blood stream if the transgene donor vector is delivered to a hepatocyte.

In some embodiments, *in vivo* transgenic animal systems are used. In some aspects, the transgenic animal may be used in model development where the transgene encodes a human IDUA or IDS protein. In some instances, the transgenic animal may be knocked out at the corresponding endogenous locus, allowing the development of an *in vivo* system where the human protein may be studied in isolation. Such transgenic models may be used for screening purposes to identify small molecules, or large biomolecules or other entities which may interact with or modify the human protein of interest.

In any of the previous embodiments, the compounds of the invention may be combined with other therapeutic agents for the treatment of subjects with MPS I or MPS II disease. In some aspects, the compositions are used in combination with methods and compositions to allow passage across the blood brain barrier. In other aspects, the compositions are used in combination with compounds known to suppress the immune response of the subject.

These and other aspects will be readily apparent to the skilled artisan in light of disclosure as a whole.

### BRIEF DESCRIPTION OF THE DRAWINGS

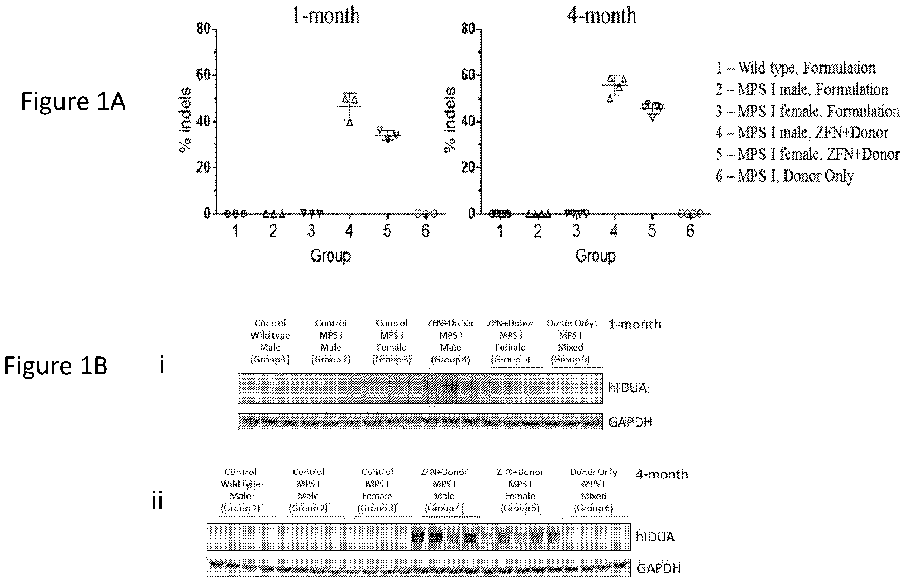

**Figures 1A and 1B** depict the results following treatment of the indicated groups of mice with the mouse albumin-specific ZFNs and the IDUA donor. In Figure 1A graphs indicate levels of ZFN activity (% indels) in the livers of mice treated with rAAV2/8 mouse surrogate ZFNs and hIDUA donor at 1 month (left panel) and 4 months (right panel) post-dosing. Genomic DNA was isolated from livers of individual mice and ZFN activity was measured by deep sequencing of the mouse albumin locus at the ZFN target site. Study group numbers are listed, and each individual symbol represents data from an individual mouse at the stated time point (at necropsy). Horizontal lines represent the mean ± standard deviation of each group. In Figure 1B, IDUA expression was measured by Western blot analysis. Surrogate mouse ZFNs and hIDUA donor were packaged as rAAV2/8 and injected IV into mice. Mice were euthanized at 1 month (panel i) or 4 months (panel ii) post-dosing and protein was extracted from the liver. Expression of HIDUA in mouse livers was determined using a human-specific IDUA antibody. GAPDH is shown as a loading control.

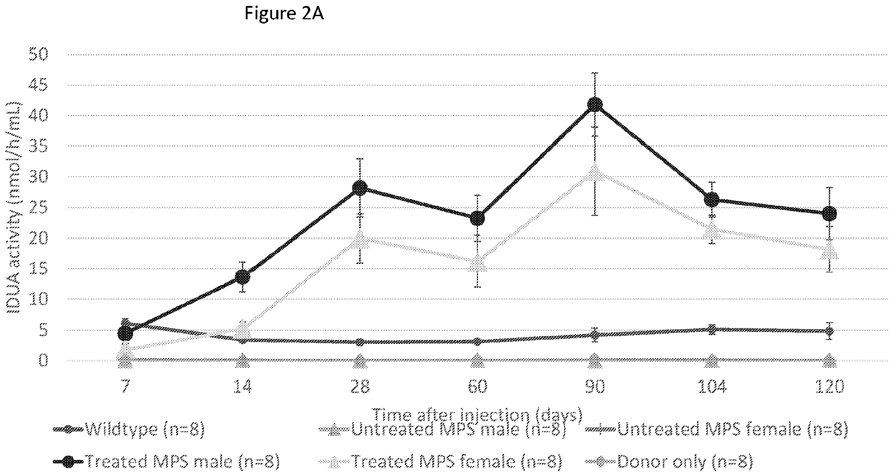

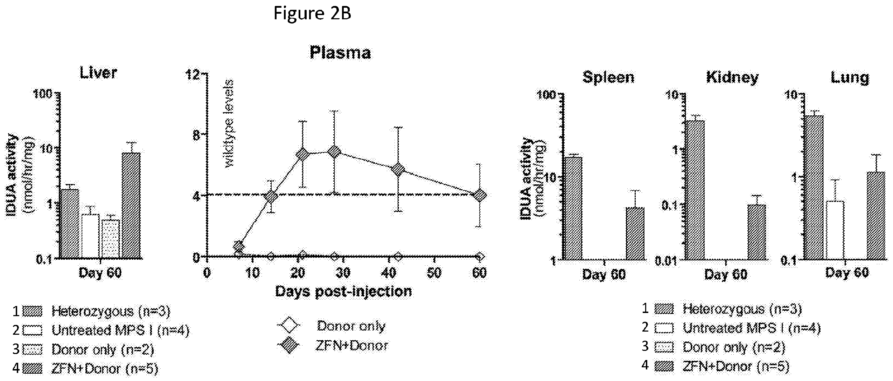

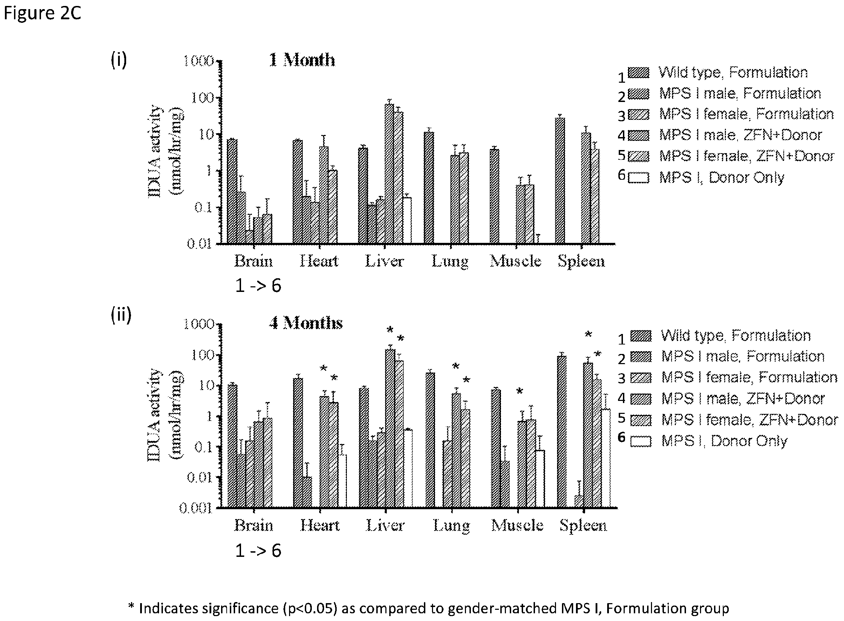

**Figures 2A through 2C** depict detectable IDUA enzymatic activity. Figure 2A is a graph depicting enzyme activity in the plasma. Surrogate mouse ZFNs and hIDUA donor were packaged as rAAV2/8 and injected IV into mice. Plasma was harvested on the indicated days and IDUA enzyme activity was determined by fluorimetric assay. Data show mean ± SD of 4-8 animals/group, depending on the time point. Two-way repeated-measures ANOVA followed by Dunnett's multiple comparison test revealed that the MPS I ZFN+Donor groups were significantly different from the wild type group from Day 14-120 for male animals, and from Day 28-120 for the female animals. Figure 2B shows the IDUA activity measured up to day 60 in the liver (left panel), plasma (middle panel), spleen (right panel), kidney (right panel) and lung (right panel). In the graphs depicting the liver, spleen, kidney and lung data, the left most bar (1) represents MPS I heterozygous mice, the next bar to the right (2) represents untreated MPS I homozygous mice, the next bar to the right (3) represents MPS I mice treated with the IDUA donor AAV only, and the bar farthest to the right (4) represents the data from MPS I mice treated with the donor AAV and the AAV comprising the albumin specific ZFN. Figure 2C has two histograms depicting the amount of IDUA activity in various tissues in mice that were sacrificed at 1 month (panel (i)) or 4 months (panel (ii)). Groups shown are: (1 - leftmost bars) wild type mice, dosed with formulation buffer; (2 - bars second from the left) MPS I males, formulation buffer; (3 - bars third from the left) MPS I females, formulation buffer; (4 - bars third from right) MPS I males, ZFN + donor; (5 - bars second from right) MPS I females, ZFN + donor; (6 - rightmost bars) MPS I, donor only.

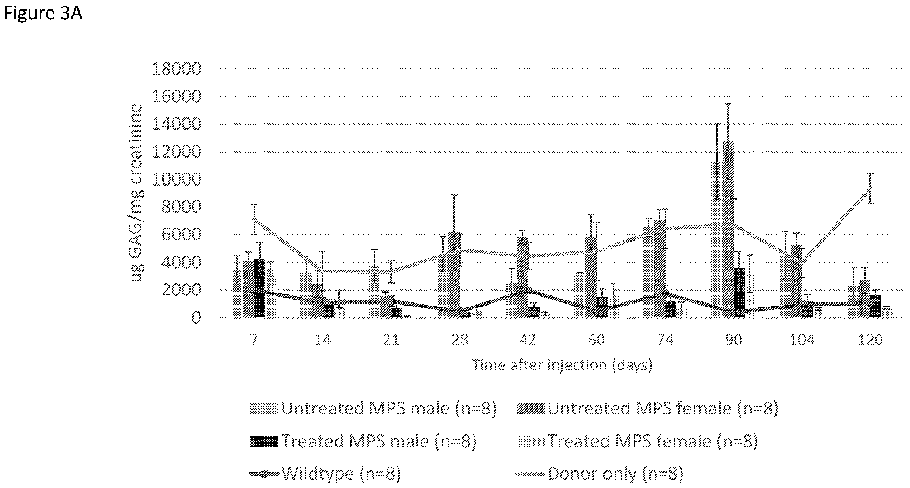

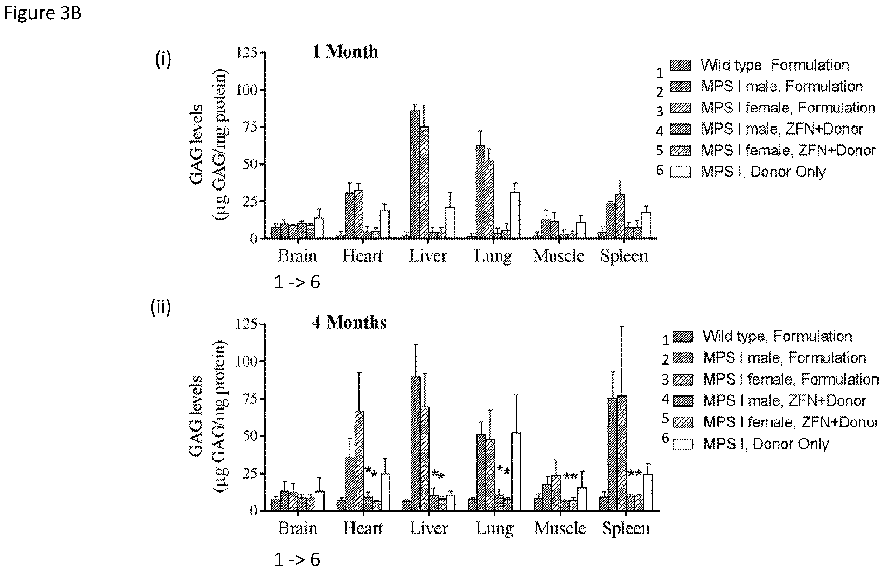

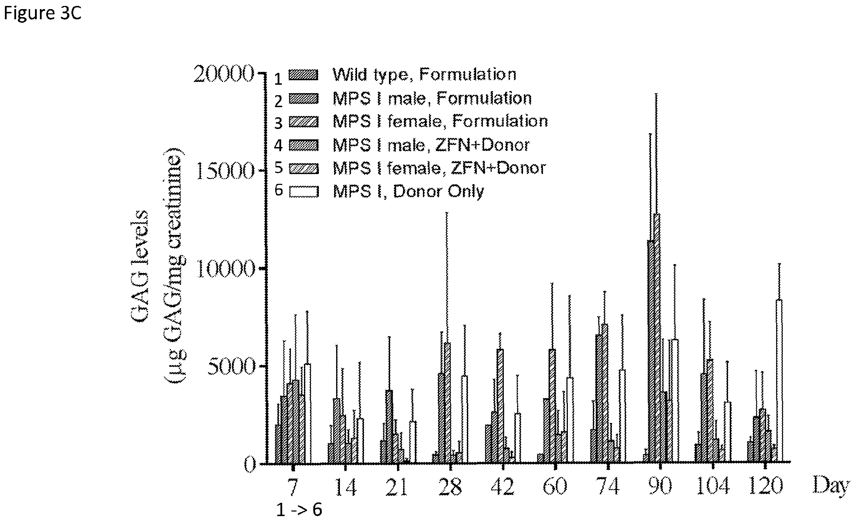

**Figures 3A through 3C** are graphs depicting a significant reduction in GAG levels in treated mice following treatment. Surrogate mouse ZFNs and hIDUA donor were packaged as rAAV2/8 and injected IV into mice. Urine was collected on the indicated days and GAG levels were determined by the Blyscan GAG assay. Figure 3A shows the GAG results in the urine following treatment. The left most bar on each group shows untreated MPS I males; the bar second from the left shows untreated MPS I females; the bar second from the right shows treated MPS I males and the right most bar shows treated MPS I females. Data show mean ± SD of 1-8 animals/group, depending on the time point. Figure 3B shows the amount of GAG detected in the various organs 1 month after treatment (Figure 3B(i)) or 4 months after treatment (Figure 3B(ii)). Groups shown are the same as above in Figure 2B and 2C. Figure 3C is a graph showing the amount of GAG detected in the urine of the various groups through 120 days post treatment. Treatment groups are the same as those in Figure 3B.

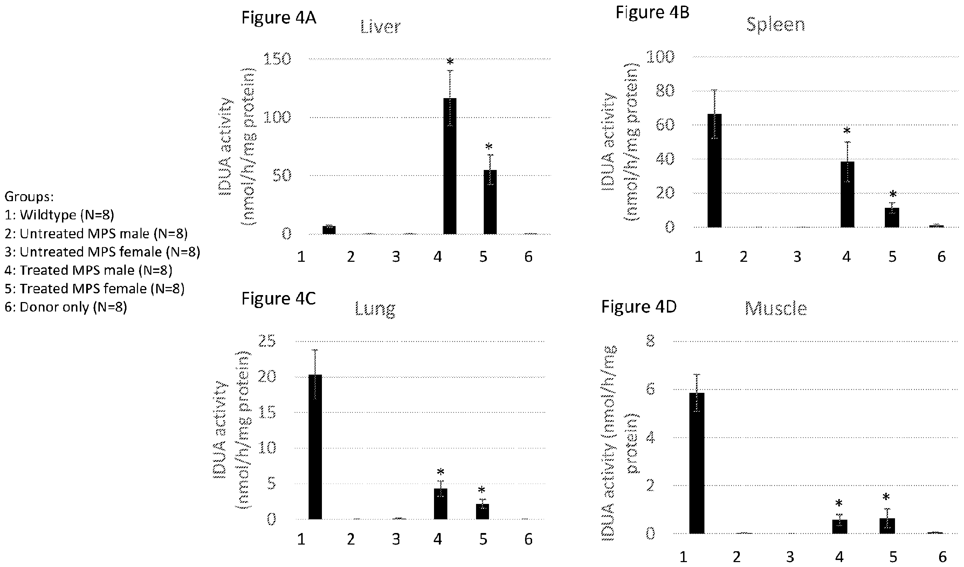

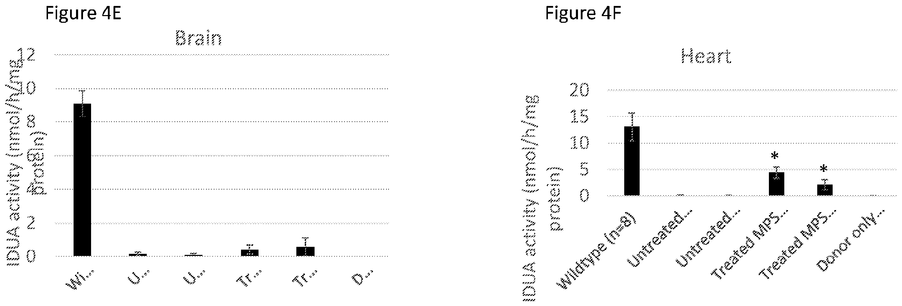

**Figures 4A through 4F** show a series of graphs depicting the amount of IDUA enzymatic activity detected in different tissues from treated mice. Surrogate mouse ZFNs and hIDUA donor were packaged as rAAV2/8 and injected IV into mice. Mice were euthanized at 4 months post-dosing and protein was extracted from the liver and other tissues. IDUA enzyme activity was determined by fluorimetric assay. Data show mean ± SD of 8 animals/group. *p<0.05 vs. respective control group (formulation-treated MPS I mice) (Mann-Whitney test). Figure 4A shows IDUA activity in liver in the indicated groups. Figure 4B shows IDUA activity in spleen in the indicated groups. Figure 4C shows IDUA activity in lung in the indicated groups. Figure 4D shows IDUA activity in muscle. Figure 4E shows IDUA activity in brain in the indicated groups and Figure 4F shows IDUA activity in heart in the indicated groups.

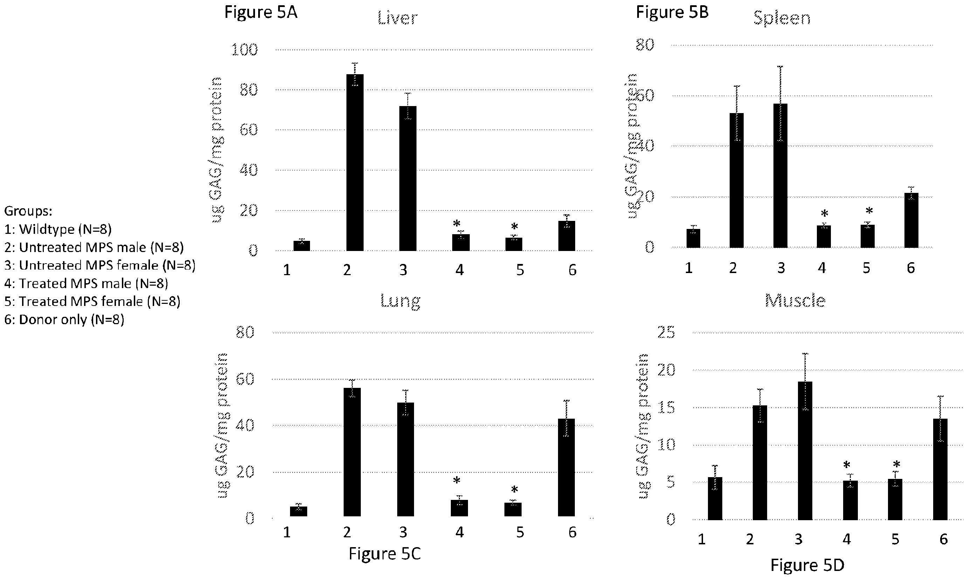

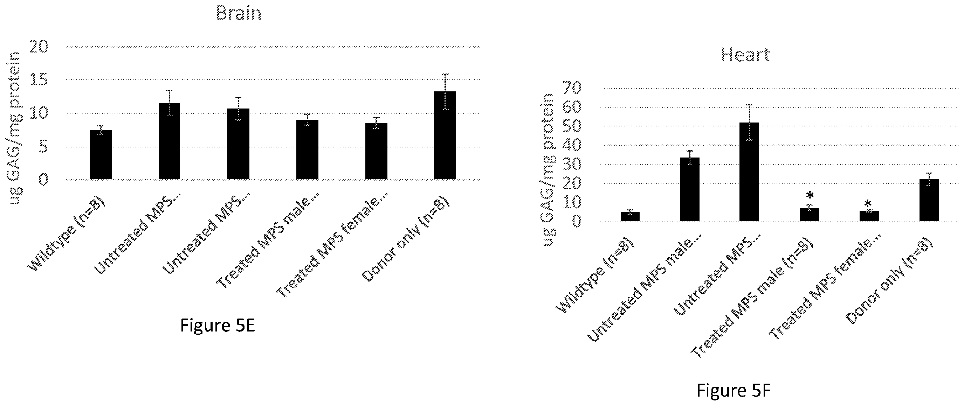

**Figures 5A through 5F** are graphs depicting significant reductions in GAG levels in treated mice following treatment. Surrogate mouse ZFNs and hIDUA donor were packaged as rAAV2/8 and injected IV into mice. Mice were euthanized at 1 and 4 months post-dosing and protein was extracted from the liver and other tissues. IDUA enzyme activity was determined by fluorimetric assay. Data show mean ± SD 8 animals/group. *p<0.05 vs. respective control group (formulation-treated MPS I mice) (Mann-Whitney test). Figure 5A shows GAG levels in liver in the indicated groups. Figure 5B shows GAG levels in spleen in the indicated groups. Figure 5C shows GAG levels in lung in the indicated groups. Figure 5D shows GAG levels in muscle. Figure 5E shows GAG levels in brain in the indicated groups and Figure 5F shows GAG levels in heart in the indicated groups.

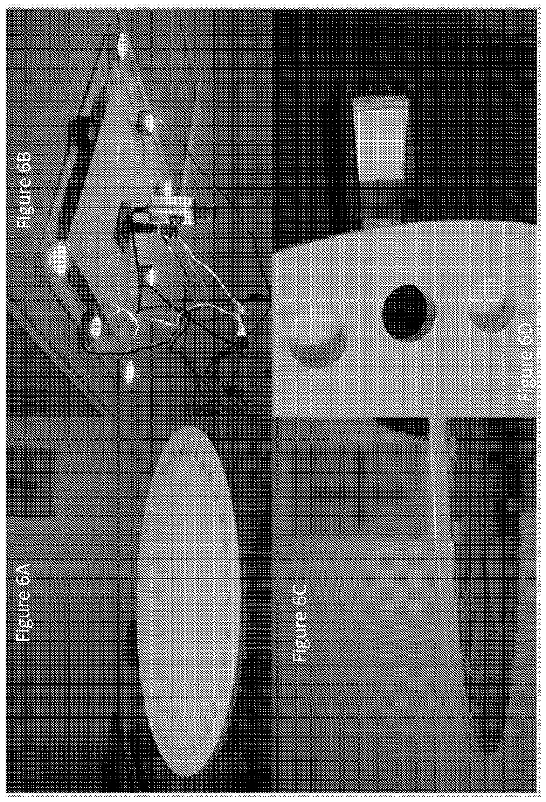

**Figures 6A through 6D** are a series of reproductions of photos showing a Barnes Maze that measured cognitive performance at 4 months post-dose. The Barnes Maze includes a platform with 40 holes (Figure 6A). Mice were placed on the platform and filmed from above with bright lighting (Figure 6B). All holes were blocked (Figure 6C) except for one, which is the escape hole (Figure 6D) that the mice seek to avoid the light.

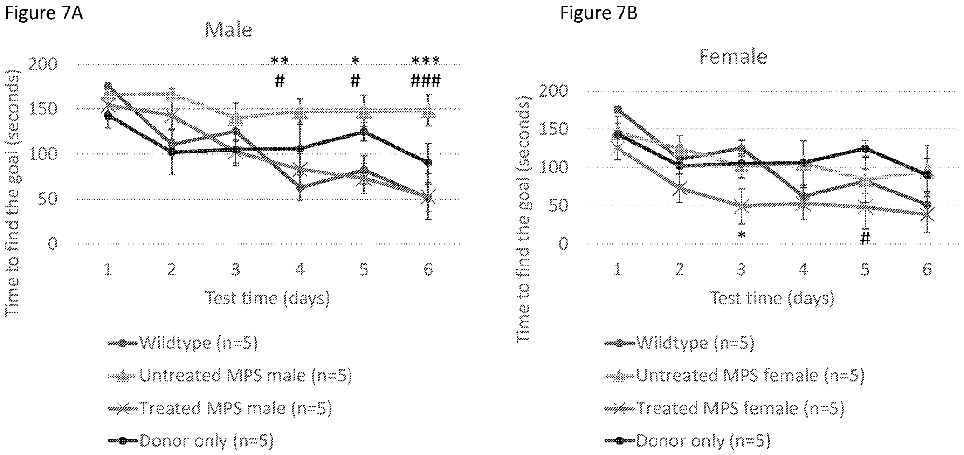

**Figures 7A and 7B** are graphs that depict the cognitive performance results using the Barnes maze test at ∼4 months post-dose. Data represent mean ± SEM of the time it took the animals to find the target escape hole (average of 4 trials each day) over 6 days of testing. Figure 7A shows results for MPS I males, Untreated, *p<0.05, **p<0.01, ***p<0.001 vs. Wild type; #p<0.05, ###p<0.001 vs. MPS I male, ZFN+ Donor; and Figure 7B shows results for MPS I females, ZFN+Donor *p<0.05 vs. Wild type; #p<0.05 vs MPS I, Donor Only (two-way repeated-measures ANOVA followed by Tukey's multiple comparisons test).

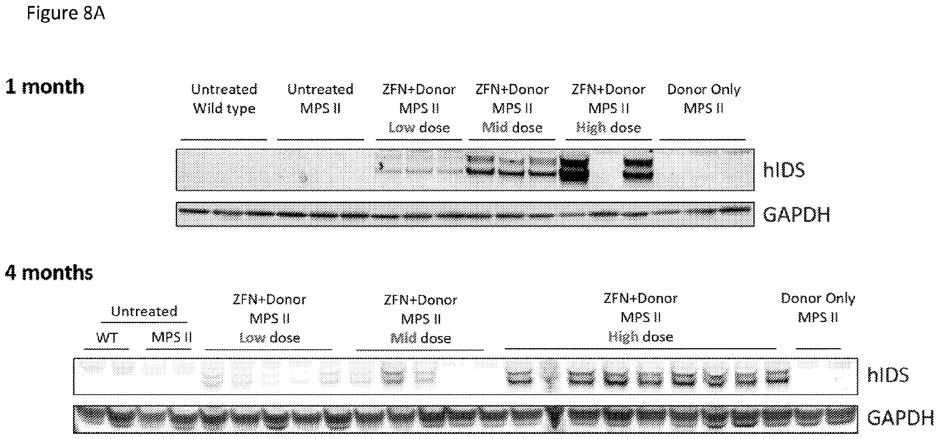

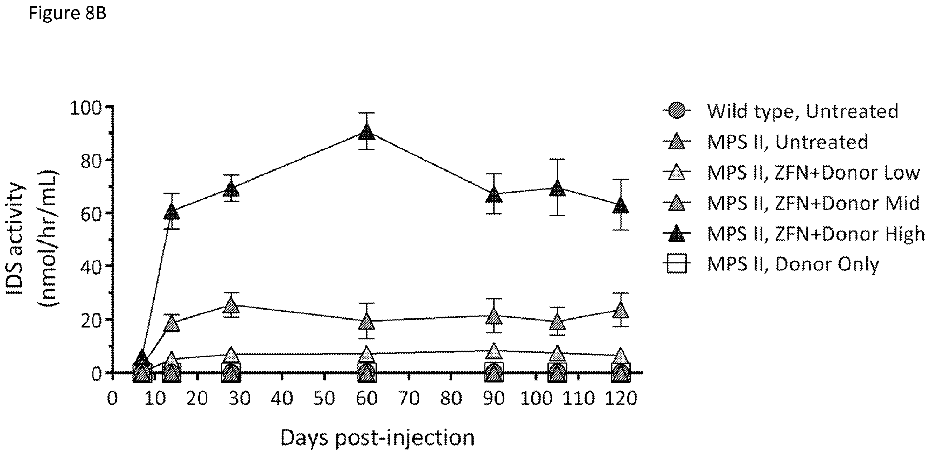

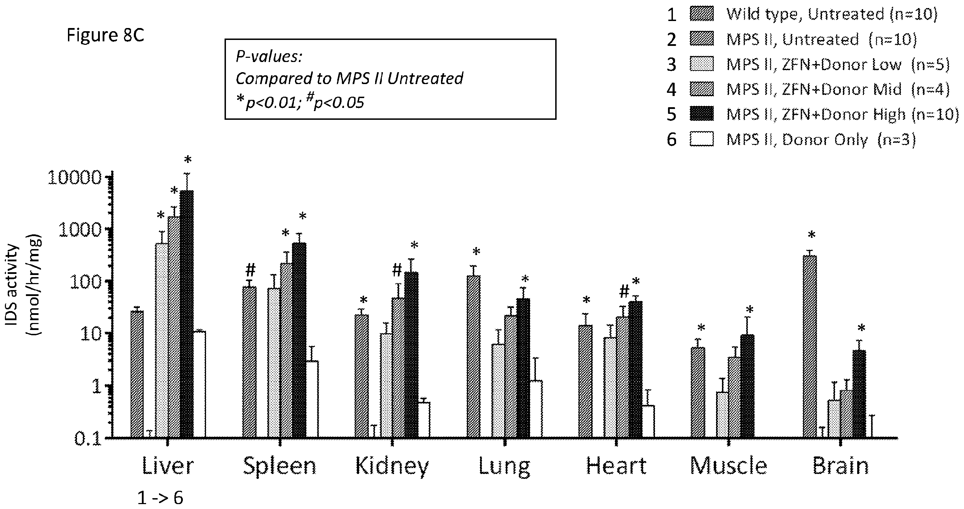

**Figures 8A through 8C** depict the expression of IDS in a mouse model of MPS II. Figure 8A is a Western blot showing expression of human IDS in mouse livers at 1 month (top panel) or 4 months (bottom panel) post-treatment. Antibodies against IDS were raised against the human protein (AF2449 from R&D Systems), with a loading control detecting mouse GAPDH (A00191 from Genscript). Figure 8B is a graph showing IDS enzymatic activity in plasma detected up to 120 days post treatment, and Figure 8C is a histogram showing IDS enzymatic activity in various tissues 4 months post dosing. Figure 8C is a graph showing groupings of the treatment groups for each tissue as follows: (1 - left most bars) Wild type, untreated mice; (2 - bars second from the left) MPS II mice, untreated; (3 - bars third from the left) MPS II mice, ZFN + donor treatment, low dose; (4- bars third from the right) MPS II mice, ZFN + donor treatment, mid dose; (5 - bars second from the right) MPS II mice, ZFN + donor treatment, high dose; (6 - right most bars) MPS II mice, donor treatment only. The order of the samples in the groups is from left to right, 1-6. Data significance is indicated.

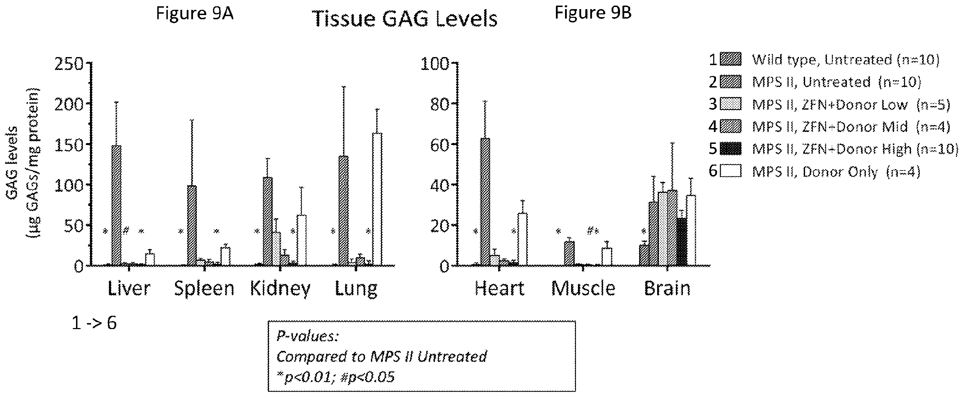

**Figures 9A and 9B** are histograms showing the GAG levels in the indicated organs of the MPS II mice 4 months post treatment. Figure 9A depicts the levels in the liver, spleen, kidney and lung while Figure 9B depicts the levels in heart, muscle and brain. Asterisks indicate data significance as compared to the MPS II untreated mice. The groups tested and order displayed is the same as for Figure 8C.

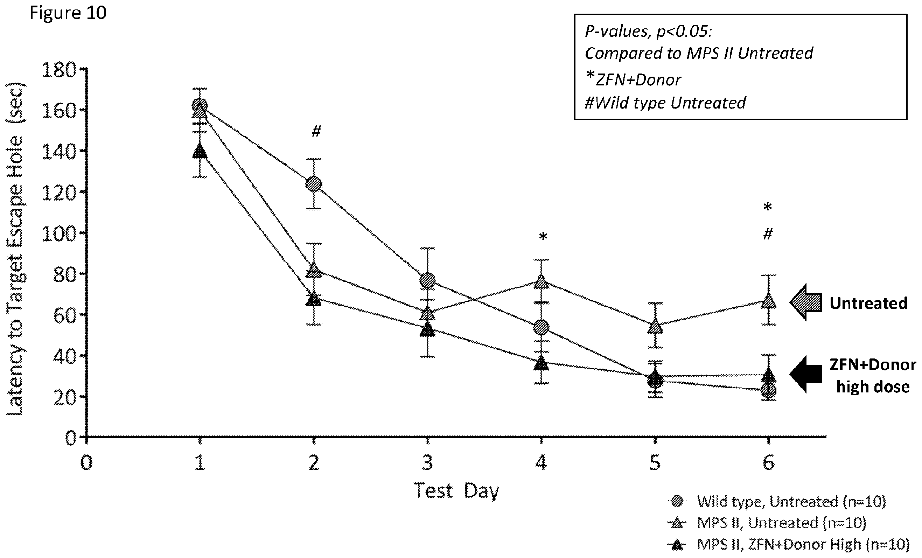

**Figure 10** is a graph showing the cognitive performance results using the Barnes maze test 4 months post-treatment. Data represent mean ± SEM of the time it took the animals to find the target escape hole (average of 4 trials each day) over 6 days of testing. Asterisks indicate data significance as compared between the MPS II untreated mice and the ZFN + Donor mice, and hashtags indicate significance as compared between the MPS II untreated mice and the wild type untreated mice.

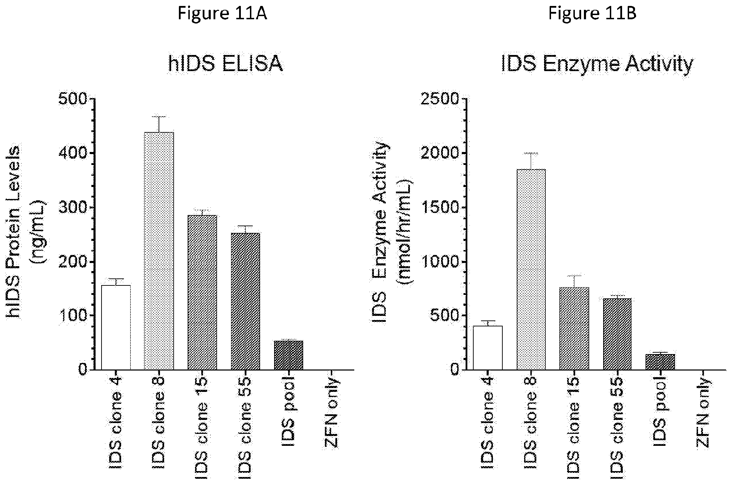

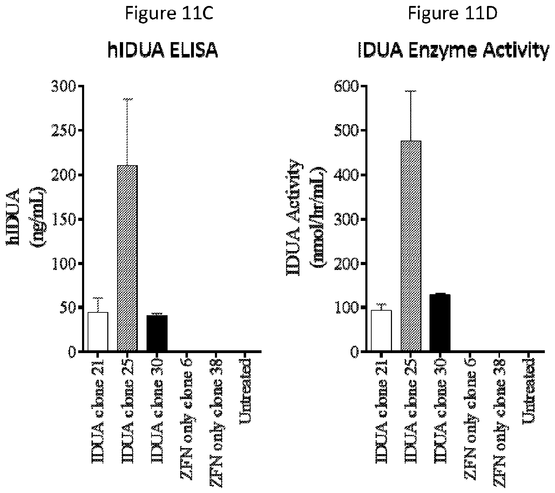

**Figures 11A through 11D** are graphs depicting IDS or IDUA activity in HepG2 cells transduced with albumin-specific ZFNs and the IDS or IDUA donor. Four subclones derived from HepG2 cells transduced with SBS#47171, SBS#47931 and SB-IDS donor (IDS), or SBS#47171 and SBS#47931 alone (ZFN only) were analyzed by hIDS ELISA (shown in Figure 11A) and IDS enzyme activity (shown in Figure 11B) assay after 72 hr supernatant accumulation. Three subclones derived from HepG2 cells transduced with SBS#47171, SBS#47931 and SB-IDUA donor (IDUA), or SBS#47171 and SBS#47931 alone (ZFN only) were analyzed by hIDUA ELISA (shown in Figure 11C) and IDUA enzyme activity (shown in Figure 11D) assay after 72 hours supernatant accumulation. Data represent means ± SD of three independent supernatants.

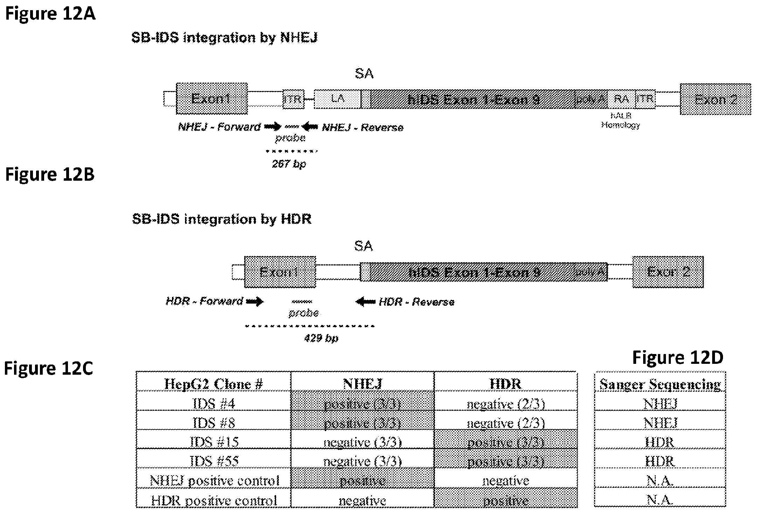

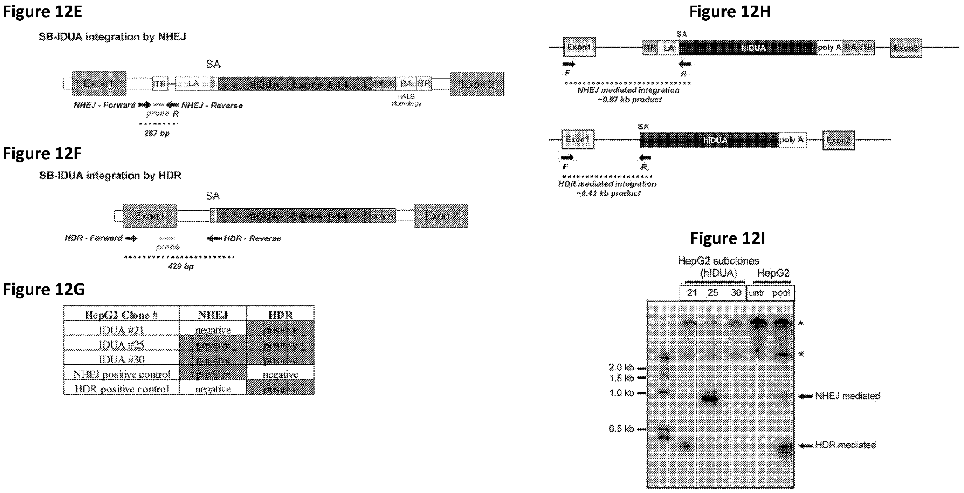

**Figure 12A through Figure 12I** indicate the types of integration of the IDS donor ("SB-IDS") or IDUA ("SB-IDUA") that may occur at the albumin locus and the results observed in the four IDS and three IDUA subclones. SB-IDS integration events at the human albumin locus by either NHEJ (Figure 12A) or HDR (Figure 12B) are shown with respective specific reagents (primers and probes) and binding sites. Figure 12C is a summary table of Taqman® data for all subclones. The numbers in parentheses indicate the number of times a clone scored positive or negative for the indicated mode of integration in the Taqman® assay out of a total of 3 assay repeats. Figure 12D is a summary table of Sanger sequencing data for all subclones, indicating the predominant mode of integration. N.A. = not applicable. SB-IDUA integration events at the human albumin locus either by NHEJ (Figures 12E and 12H) or HDR (Figure 12F) and also indicate the size of the expected bands. Figure 12G is a summary table of Taqman® data for the indicated subclones. Figure 12I is a reproduction of a gel confirming HDR integration of IDUA for clone 21, NHEJ for clone 25 and NHEJ for clone 30.

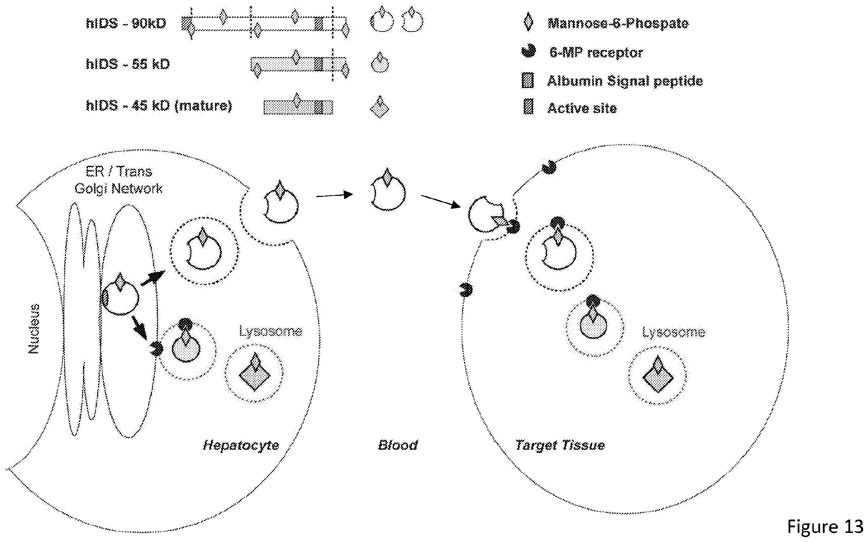

**Figure 13** is a schematic showing the process in which the IDS is produced in the transduced liver and then taken up and subsequently processed into the final mature protein.

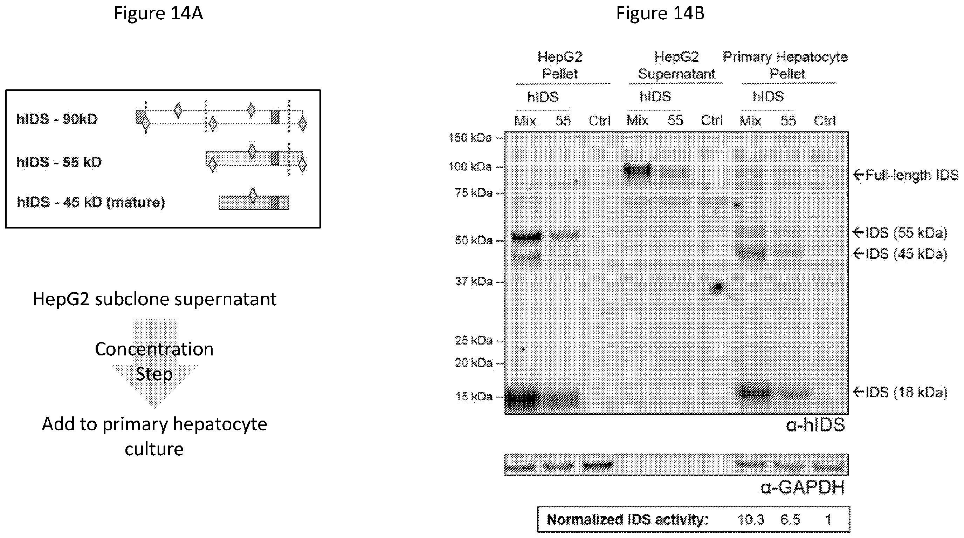

**Figures 14A and 14B** show the uptake of the secreted IDS from the HepG2 subclones in primary hepatocytes. Figure 14A shows the different protein forms as the IDS protein is matured and shows the process for adding the HepG2 supernatant to the primary hepatocyte cultures. Figure 14B shows a Western blot depicting the forms of IDS detected in the HepG2 clones ("Mix" and 55) supernatant and pellets, and the forms detected in the primary hepatocyte pellet, illustrating the processing of the full-length polypeptide (90 kDa) during the production of the mature form (45 kDa). A loading control of a housekeeping protein (a-GAPDH) is also shown as well as the relative IDS activity in the primary hepatocyte pellets following IDS update from the supernatant.

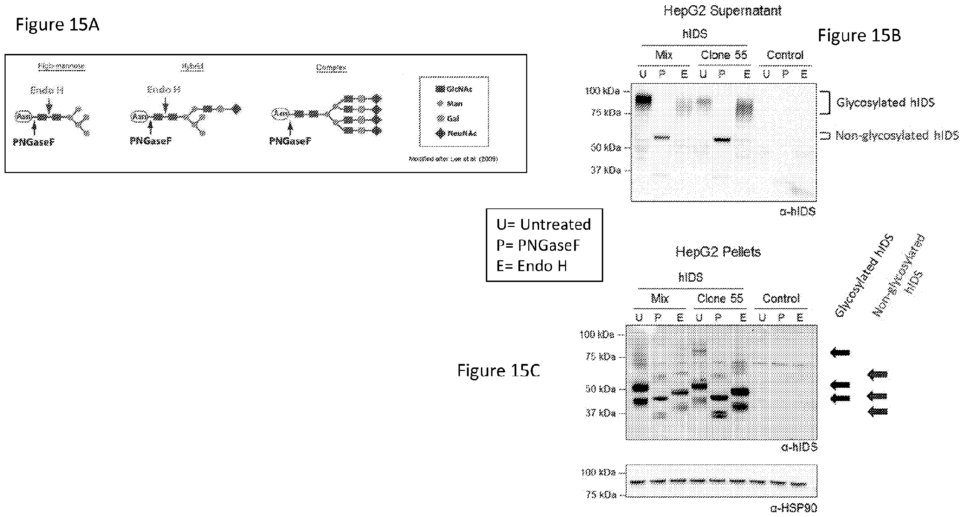

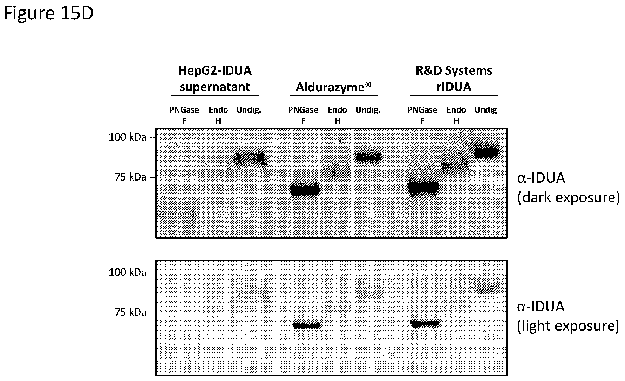

**Figures 15A through 15D** illustrate the investigation into the glycosylation patterns on the IDS or IDUA produced in the HepG2 subclones. Figure 15A shows the different kinds of glycosylation patterns and the substrate specificities of glycosidases used in deglycosylation (modified from Lee et al (2009) Nat Protoc. 4(4):592-604). Figures 15B and 15C are Western blots using an anti-IDS antibody investigating the IDS protein produced in the HepG2 clones where Figure 15B shows the protein found in the cell supernatant while Figure 15C shows the protein in the cell pellets following treatment with the glycosidases PNGaseF and Endo H. Treatment with PNGase F removed all oligosaccharide chains, which results in "Non-glycosylated hIDS" as indicated. For the HepG2 pellet Western blot, HSP90 is shown as a loading control 'Mix' = a mixed HepG2 IDS population consisting of 2 different IDS subclones. 'Clone 55' = HepG2 IDS subclone #55. 'Control' = a HepG2 subclone with IDUA, instead of IDS, integrated at the Albumin locus as a negative control. 'kDa' = kilodaltons (molecular weight marker). Figure 15D shows Western blots (top panel shows dark exposure and bottom panel shows light exposure) using an anti-IDUA antibody to investigate the glycosylation patterns of the hIDUA produced in comparison with commercially available hIDUA (Alsurazyme® and R&D Systems IDUA). As before, the enzymes were treated with PNGase F, Endo H or left undigested.

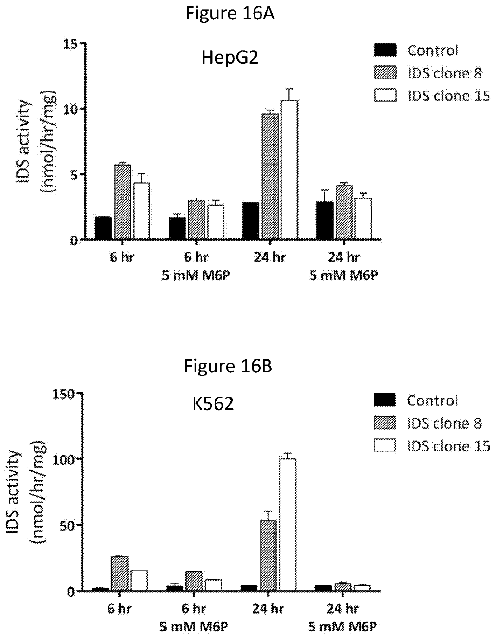

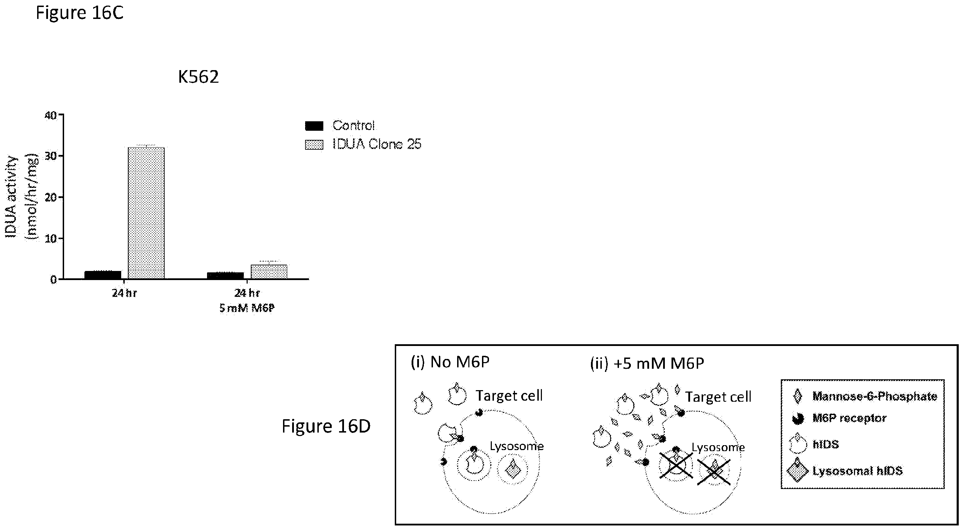

**Figure 16A through 16D** depict the effect of added mannose 6-phosphase (M6P) on uptake of IDS by either HepG2 cells (Figure 16A) or K562 cells (Figure 16B) and a graph of the uptake of IDUA produced by K562 cells (Figure 16C). Figure 16D is a schematic showing the effect on uptake of treatment of a target cell with mannose-6-phosphate. The data depicted illustrates cells treated either with M6P or left untreated. In Figures 16A and 16B, each three-membered group, the samples shown, from left to right are as follows: left most bar- control comprising treatment with conditioned media from untreated HepG2 cells; middle bar- cells treated with conditioned media from the HepG2-IDS clone 8; right bar- cells treated with conditioned media from the HepG2-IDS clone 15. At each time point, cells treated with the M6P are indicated. Following the indicated time period, the cells were pelleted and then assayed for IDS activity. Comparison of the two conditions illustrates that the M6P inhibited the uptake of the glycosylated IDS protein. Figure 16C is a depicting IDUA uptake by K562 cells cultured in conditioned media obtained from untreated HepG2 cells or HepG2 cells with the IDUA donor (IDUA clone number 25). Figure 16D is a schematic showing the effect of treatment of the cells with mannose-6-phosphate (M6P), illustrating how the M6P treatment in Figures 16A-16C blocks enzyme uptake, decreasing the amount of IDUA detectable in the cells.

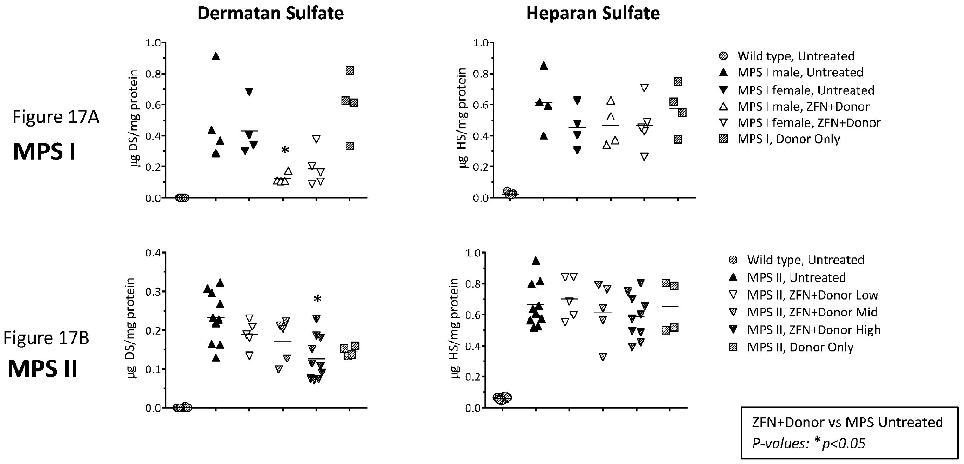

**Figures 17A and 17B** depict analysis of dermatan sulfate (left panels) and heparan sulfate (right panels) levels in the brains of treated and untreated wild type and MPS I & II mice. Figure 17A corresponds to the mice analyzed in Figure 3B(ii), leftmost set of columns. Figure 17B corresponds to the mice analyzed in Figure 9B, rightmost set of columns.

### DETAILED DESCRIPTION

Disclosed herein are methods and compositions for treating and/or preventing MPS I or MPS II disease, in particular, methods and compositions for insertion of a gene encoding a protein that is lacking or insufficiently expressed in the subject with MPS I or MPS II disease such that the gene is expressed *in vivo* in one or more cells and/or tissues of the subject. The IDUA or IDS gene is expressed from a liver cell as well as secreted from the liver where it is functional in secondary cells and tissues (e.g., spleen, lung, heart, muscle, brain, etc.).

Thus, the compositions of the invention can be used to express from a transgene therapeutically beneficial MPS I and/or MPS II proteins (IDUA or IDS) from the highly expressed albumin locus to replace the enzyme that is defective in MPS I or MPS II disease. Additionally, the invention provides compositions for treatment (including the alleviation of one or more symptoms) of MPS I and/or MPS II disease by insertion of the transgene sequences into a highly-expressed albumin locus in liver cells.

### General

Practice of the methods, as well as preparation and use of the compositions disclosed herein employ, unless otherwise indicated, conventional techniques in molecular biology, biochemistry, chromatin structure and analysis, computational chemistry, cell culture, recombinant DNA and related fields as are within the skill of the art. These techniques are fully explained in the literature. *See,* for example, Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, "Chromatin" (P.M. Wassarman and A. P. Wolffe, eds.), Academic Press, San Diego, 1999; and METHODS IN MOLECULAR BIOLOGY, Vol. 119, "Chromatin Protocols" (P.B. Becker, ed.) Humana Press, Totowa, 1999.

### Definitions

The terms "nucleic acid," "polynucleotide," and "oligonucleotide" are used interchangeably and refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties (*e.g*., phosphorothioate backbones). In general, an analogue of a particular nucleotide has the same base-pairing specificity; *i.e.,* an analogue of A will base-pair with T.

The terms "polypeptide," "peptide" and "protein" are used interchangeably to refer to a polymer of amino acid residues. The term also applies to amino acid polymers in which one or more amino acids are chemical analogues or modified derivatives of corresponding naturally-occurring amino acids.

"Binding" refers to a sequence-specific, non-covalent interaction between macromolecules (*e.g*., between a protein and a nucleic acid). Not all components of a binding interaction need be sequence-specific (*e.g.,* contacts with phosphate residues in a DNA backbone), as long as the interaction as a whole is sequence-specific. Such interactions are generally characterized by a dissociation constant (K_{d}) of 10⁻⁶ M⁻¹ or lower. "Affinity" refers to the strength of binding: increased binding affinity being correlated with a lower K_{d}.

A "binding protein" is a protein that is able to bind non-covalently to another molecule. A binding protein can bind to, for example, a DNA molecule (a DNA-binding protein), an RNA molecule (an RNA-binding protein) and/or a protein molecule (a protein-binding protein). In the case of a protein-binding protein, it can bind to itself (to form homodimers, homotrimers, *etc.*) and/or it can bind to one or more molecules of a different protein or proteins. A binding protein can have more than one type of binding activity. For example, zinc finger proteins have DNA-binding, RNA-binding and protein-binding activity.

A "zinc finger DNA binding protein" (or binding domain) is a protein, or a domain within a larger protein, that binds DNA in a sequence-specific manner through one or more zinc fingers, which are regions of amino acid sequence within the binding domain whose structure is stabilized through coordination of a zinc ion. The term zinc finger DNA binding protein is often abbreviated as zinc finger protein or ZFP.

A "TALE DNA binding domain" or "TALE" is a polypeptide comprising one or more TALE repeat domains/units. The repeat domains are involved in binding of the TALE to its cognate target DNA sequence. A single "repeat unit" (also referred to as a "repeat") is typically 33-35 amino acids in length and exhibits at least some sequence homology with other TALE repeat sequences within a naturally occurring TALE protein. *See, e.g.,* U.S. Patent No. 8,586,526.

Zinc finger and TALE binding domains can be "engineered" to bind to a predetermined nucleotide sequence, for example via engineering (altering one or more amino acids) of the recognition helix region of a naturally occurring zinc finger or TALE protein. Therefore, engineered DNA binding proteins (zinc fingers or TALEs) are proteins that are non-naturally occurring. Non-limiting examples of methods for engineering DNA-binding proteins are design and selection. A designed DNA binding protein is a protein not occurring in nature whose design/composition results principally from rational criteria. Rational criteria for design include application of substitution rules and computerized algorithms for processing information in a database storing information of existing ZFP and/or TALE designs and binding data. See, for example, U.S. Patent Nos. 8,568,526; 6,140,081; 6,453,242; and 6,534,261; see also WO 98/53058; WO 98/53059; WO 98/53060; WO 02/016536 and WO 03/016496.

A "selected" zinc finger protein or TALE is a protein not found in nature whose production results primarily from an empirical process such as phage display, interaction trap or hybrid selection. See *e.g.,* U.S. Patent Nos. 8,586,526; 5,789,538; 5,925,523; 6,007,988; 6,013,453; 6,200,759; and WO 95/19431; WO 96/06166; WO 98/53057; WO 98/54311; WO 00/27878; WO 01/60970; WO 01/88197 and WO 02/099084.

"Recombination" refers to a process of exchange of genetic information between two polynucleotides. For the purposes of this disclosure, "homologous recombination (HR)" refers to the specialized form of such exchange that takes place, for example, during repair of double-strand breaks in cells via homology-directed repair mechanisms. This process requires nucleotide sequence homology, uses a "donor" molecule to template repair of a "target" molecule (*i.e.,* the one that experienced the double-strand break), and is variously known as "non-crossover gene conversion" or "short tract gene conversion," because it leads to the transfer of genetic information from the donor to the target. Without wishing to be bound by any particular theory, such transfer can involve mismatch correction of heteroduplex DNA that forms between the broken target and the donor, and/or "synthesis-dependent strand annealing," in which the donor is used to re-synthesize genetic information that will become part of the target, and/or related processes. Such specialized HR often results in an alteration of the sequence of the target molecule such that part or all of the sequence of the donor polynucleotide is incorporated into the target polynucleotide.

One or more targeted nucleases as described herein create a double-stranded break in the target sequence (*e.g*., cellular chromatin) at a predetermined site, and a "donor" polynucleotide, having homology to the nucleotide sequence in the region of the break, can be introduced into the cell. The presence of the double-stranded break has been shown to facilitate integration of the donor sequence. The donor sequence may be physically integrated or, alternatively, the donor polynucleotide is used as a template for repair of the break via homologous recombination, resulting in the introduction of all or part of the nucleotide sequence as in the donor into the cellular chromatin. Thus, a first sequence in cellular chromatin can be altered and, in certain embodiments, can be converted into a sequence present in a donor polynucleotide. Thus, the use of the terms "replace" or "replacement" can be understood to represent replacement of one nucleotide sequence by another, (*i.e.,* replacement of a sequence in the informational sense), and does not necessarily require physical or chemical replacement of one polynucleotide by another.

Additional pairs of zinc-finger or TALEN proteins can be used for additional double-stranded cleavage of additional target sites within the cell.

For targeted recombination and/or replacement and/or alteration of a sequence in a region of interest in cellular chromatin, a chromosomal sequence is altered by homologous recombination with an exogenous "donor" nucleotide sequence. Such homologous recombination is stimulated by the presence of a double-stranded break in cellular chromatin, if sequences homologous to the region of the break are present.

The first nucleotide sequence (the "donor sequence") can contain sequences that are homologous, but not identical, to genomic sequences in the region of interest, thereby stimulating homologous recombination to insert a non-identical sequence in the region of interest. Thus, in certain embodiments, portions of the donor sequence that are homologous to sequences in the region of interest exhibit between about 80 to 99% (or any integer therebetween) sequence identity to the genomic sequence that is replaced. In other embodiments, the homology between the donor and genomic sequence is higher than 99%, for example if only 1 nucleotide differs as between donor and genomic sequences of over 100 contiguous base pairs. In certain cases, a non-homologous portion of the donor sequence can contain sequences not present in the region of interest, such that new sequences are introduced into the region of interest. In these instances, the non-homologous sequence is generally flanked by sequences of 50-1,000 base pairs (or any integral value therebetween) or any number of base pairs greater than 1,000, that are homologous or identical to sequences in the region of interest. In other embodiments, the donor sequence is non-homologous to the first sequence, and is inserted into the genome by non-homologous recombination mechanisms.

Furthermore, targeted integration as described herein can also be used to integrate one or more exogenous sequences. The exogenous nucleic acid sequence can comprise, for example, one or more genes or cDNA molecules, or any type of coding or non-coding sequence, as well as one or more control elements. In addition, the exogenous nucleic acid sequence may produce one or more RNA molecules (*e.g*., small hairpin RNAs (shRNAs), inhibitory RNAs (RNAis), microRNAs (miRNAs), *etc*.)*.*

"Cleavage" refers to the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends. In certain embodiments, fusion polypeptides are used for targeted double-stranded DNA cleavage.

A "cleavage half-domain" is a polypeptide sequence which, in conjunction with a second polypeptide (either identical or different) forms a complex having cleavage activity (preferably double-strand cleavage activity). The terms "first and second cleavage half-domains;" "+ and - cleavage half-domains" and "right and left cleavage half-domains" are used interchangeably to refer to pairs of cleavage half-domains that dimerize.

An "engineered cleavage half-domain" is a cleavage half-domain that has been modified so as to form obligate heterodimers with another cleavage half-domain (*e.g.,* another engineered cleavage half-domain). *See,* U.S. Patent Nos. 7,888,121; 7,914,796; 8,034,598 and 8,823,618.

The term "sequence" refers to a nucleotide sequence of any length, which can be DNA or RNA; can be linear, circular or branched and can be either single-stranded or double stranded. The term "donor sequence" refers to a nucleotide sequence that is inserted into a genome. A donor sequence can be of any length, for example between 2 and 10,000 nucleotides in length (or any integer value therebetween or thereabove), preferably between about 100 and 1,000 nucleotides in length (or any integer therebetween), more preferably between about 200 and 500 nucleotides in length.

A "disease associated gene" is one that is defective in some manner in a monogenic disease. Non-limiting examples of monogenic diseases include severe combined immunodeficiency, cystic fibrosis, lysosomal storage diseases (*e.g*. Gaucher's, Hurler's Hunter's, Fabry's, Neimann-Pick, Tay-Sach's etc), sickle cell anemia, and thalassemia.

The "blood brain barrier" is a highly selective permeability barrier that separates the circulating blood from the brain in the central nervous system. The blood brain barrier is formed by brain endothelial cells which are connected by tight junctions in the CNS vessels that restrict the passage of blood solutes. The blood brain barrier has long been thought to prevent the uptake of large molecule therapeutics and prevent the uptake of most small molecule therapeutics (Pardridge (2005) NeuroRx 2(1): 3-14).

"Chromatin" is the nucleoprotein structure comprising the cellular genome. Cellular chromatin comprises nucleic acid, primarily DNA, and protein, including histones and non-histone chromosomal proteins. The majority of eukaryotic cellular chromatin exists in the form of nucleosomes, wherein a nucleosome core comprises approximately 150 base pairs of DNA associated with an octamer comprising two each of histones H2A, H2B, H3 and H4; and linker DNA (of variable length depending on the organism) extends between nucleosome cores. A molecule of histone HI is generally associated with the linker DNA. For the purposes of the present disclosure, the term "chromatin" is meant to encompass all types of cellular nucleoprotein, both prokaryotic and eukaryotic. Cellular chromatin includes both chromosomal and episomal chromatin.

A "chromosome," is a chromatin complex comprising all or a portion of the genome of a cell. The genome of a cell is often characterized by its karyotype, which is the collection of all the chromosomes that comprise the genome of the cell. The genome of a cell can comprise one or more chromosomes.

An "episome" is a replicating nucleic acid, nucleoprotein complex or other structure comprising a nucleic acid that is not part of the chromosomal karyotype of a cell. Examples of episomes include plasmids and certain viral genomes.

A "target site" or "target sequence" is a nucleic acid sequence that defines a portion of a nucleic acid to which a binding molecule will bind, provided sufficient conditions for binding exist.

An "exogenous" molecule is a molecule that is not normally present in a cell, but can be introduced into a cell by one or more genetic, biochemical or other methods. "Normal presence in the cell" is determined with respect to the particular developmental stage and environmental conditions of the cell. Thus, for example, a molecule that is present only during embryonic development of muscle is an exogenous molecule with respect to an adult muscle cell. Similarly, a molecule induced by heat shock is an exogenous molecule with respect to a non-heat-shocked cell. An exogenous molecule can comprise, for example, a functioning version of a malfunctioning endogenous molecule or a malfunctioning version of a normally-functioning endogenous molecule.

An exogenous molecule can be, among other things, a small molecule, such as is generated by a combinatorial chemistry process, or a macromolecule such as a protein, nucleic acid, carbohydrate, lipid, glycoprotein, lipoprotein, polysaccharide, any modified derivative of the above molecules, or any complex comprising one or more of the above molecules. Nucleic acids include DNA and RNA, can be single- or double-stranded; can be linear, branched or circular; and can be of any length. Nucleic acids include those capable of forming duplexes, as well as triplex-forming nucleic acids. See, for example, U.S. Patent Nos. 5,176,996 and 5,422,251. Proteins include, but are not limited to, DNA-binding proteins, transcription factors, chromatin remodeling factors, methylated DNA binding proteins, polymerases, methylases, demethylases, acetylases, deacetylases, kinases, phosphatases, integrases, recombinases, ligases, topoisomerases, gyrases and helicases.

An exogenous molecule can be the same type of molecule as an endogenous molecule, *e.g.,* an exogenous protein or nucleic acid. For example, an exogenous nucleic acid can comprise an infecting viral genome, a plasmid or episome introduced into a cell, or a chromosome that is not normally present in the cell. Methods for the introduction of exogenous molecules into cells are known to those of skill in the art and include, but are not limited to, lipid-mediated transfer (*i.e.,* liposomes, including neutral and cationic lipids), electroporation, direct injection, cell fusion, particle bombardment, calcium phosphate co-precipitation, DEAE-dextran-mediated transfer and viral vector-mediated transfer. An exogenous molecule can also be the same type of molecule as an endogenous molecule but derived from a different species than the cell is derived from. For example, a human nucleic acid sequence may be introduced into a cell line originally derived from a mouse or hamster.

By contrast, an "endogenous" molecule is one that is normally present in a particular cell at a particular developmental stage under particular environmental conditions. For example, an endogenous nucleic acid can comprise a chromosome, the genome of a mitochondrion, chloroplast or other organelle, or a naturally-occurring episomal nucleic acid. Additional endogenous molecules can include proteins, for example, transcription factors and enzymes.

A "fusion" molecule is a molecule in which two or more subunit molecules are linked, preferably covalently. The subunit molecules can be the same chemical type of molecule, or can be different chemical types of molecules. Examples of fusion molecules include, but are not limited to, fusion proteins (for example, a fusion between a protein DNA-binding domain and a cleavage domain), fusions between a polynucleotide DNA-binding domain (*e.g*., sgRNA) operatively associated with a cleavage domain, and fusion nucleic acids (for example, a nucleic acid encoding the fusion protein).

Expression of a fusion protein in a cell can result from delivery of the fusion protein to the cell or by delivery of a polynucleotide encoding the fusion protein to a cell, wherein the polynucleotide is transcribed, and the transcript is translated, to generate the fusion protein. Trans-splicing, polypeptide cleavage and polypeptide ligation can also be involved in expression of a protein in a cell. Methods for polynucleotide and polypeptide delivery to cells are presented elsewhere in this disclosure.

A "gene," for the purposes of the present disclosure, includes a DNA region encoding a gene product (see *infra*), as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

"Gene expression" refers to the conversion of the information, contained in a gene, into a gene product. A gene product can be the direct transcriptional product of a gene (*e.g.,* mRNA, tRNA, rRNA, antisense RNA, ribozyme, structural RNA or any other type of RNA) or a protein produced by translation of an mRNA. Gene products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

"Modulation" of gene expression refers to a change in the activity of a gene. Modulation of expression can include, but is not limited to, gene activation and gene repression. Genome editing (*e.g*., cleavage, alteration, inactivation, random mutation) can be used to modulate expression. Gene inactivation refers to any reduction in gene expression as compared to a cell that does not include a ZFP or TALEN as described herein. Thus, gene inactivation may be partial or complete.

A "region of interest" is any region of cellular chromatin, such as, for example, a gene or a non-coding sequence within or adjacent to a gene, in which it is desirable to bind an exogenous molecule. Binding can be for the purposes of targeted DNA cleavage and/or targeted recombination. A region of interest can be present in a chromosome, an episome, an organellar genome (*e.g*., mitochondrial, chloroplast), or an infecting viral genome, for example. A region of interest can be within the coding region of a gene, within transcribed non-coding regions such as, for example, leader sequences, trailer sequences or introns, or within non-transcribed regions, either upstream or downstream of the coding region. A region of interest can be as small as a single nucleotide pair or up to 2,000 nucleotide pairs in length, or any integral value of nucleotide pairs.

"Eukaryotic" cells include, but are not limited to, fungal cells (such as yeast), plant cells, animal cells, mammalian cells and human cells (*e.g.,* T-cells).

"Red Blood Cells" (RBCs) or erythrocytes are terminally differentiated cells derived from hematopoietic stem cells. They lack a nuclease and most cellular organelles. RBCs contain hemoglobin to carry oxygen from the lungs to the peripheral tissues. In fact, 33% of an individual RBC is hemoglobin. They also carry CO2 produced by cells during metabolism out of the tissues and back to the lungs for release during exhale. RBCs are produced in the bone marrow in response to blood hypoxia which is mediated by release of erythropoietin (EPO) by the kidney. EPO causes an increase in the number of proerythroblasts and shortens the time required for full RBC maturation. After approximately 120 days, since the RBC do not contain a nucleus or any other regenerative capabilities, the cells are removed from circulation by either the phagocytic activities of macrophages in the liver, spleen and lymph nodes (∼90%) or by hemolysis in the plasma (∼10%). Following macrophage engulfment, chemical components of the RBC are broken down within vacuoles of the macrophages due to the action of lysosomal enzymes.

"Secretory tissues" are those tissues in an animal that secrete products out of the individual cell into a lumen of some type which are typically derived from epithelium. Examples of secretory tissues that are localized to the gastrointestinal tract include the cells that line the gut, the pancreas, and the gallbladder. Other secretory tissues include the liver, tissues associated with the eye and mucous membranes such as salivary glands, mammary glands, the prostate gland, the pituitary gland and other members of the endocrine system. Additionally, secretory tissues include individual cells of a tissue type which are capable of secretion.

The terms "operative linkage" and "operatively linked" (or "operably linked") are used interchangeably with reference to a juxtaposition of two or more components (such as sequence elements), in which the components are arranged such that both components function normally and allow the possibility that at least one of the components can mediate a function that is exerted upon at least one of the other components. By way of illustration, a transcriptional regulatory sequence, such as a promoter, is operatively linked to a coding sequence if the transcriptional regulatory sequence controls the level of transcription of the coding sequence in response to the presence or absence of one or more transcriptional regulatory factors. A transcriptional regulatory sequence is generally operatively linked in *cis* with a coding sequence, but need not be directly adjacent to it. For example, an enhancer is a transcriptional regulatory sequence that is operatively linked to a coding sequence, even though they are not contiguous.

With respect to fusion polypeptides, the term "operatively linked" can refer to the fact that each of the components performs the same function in linkage to the other component as it would if it were not so linked. For example, with respect to a fusion polypeptide in which a ZFP or TALE DNA-binding domain is fused to an activation domain, the ZFP or TALE DNA-binding domain and the activation domain are in operative linkage if, in the fusion polypeptide, the ZFP or TALE DNA-binding domain portion is able to bind its target site and/or its binding site, while the activation domain is able to up-regulate gene expression. When a fusion polypeptide in which a ZFP or TALE DNA-binding domain is fused to a cleavage domain, the ZFP or TALE DNA-binding domain and the cleavage domain are in operative linkage if, in the fusion polypeptide, the ZFP or TALE DNA-binding domain portion is able to bind its target site and/or its binding site, while the cleavage domain is able to cleave DNA in the vicinity of the target site.

A "functional fragment" of a protein, polypeptide or nucleic acid is a protein, polypeptide or nucleic acid whose sequence is not identical to the full-length protein, polypeptide or nucleic acid, yet retains the same function as the full-length protein, polypeptide or nucleic acid. A functional fragment can possess more, fewer, or the same number of residues as the corresponding native molecule, and/or can contain one or more amino acid or nucleotide substitutions. Methods for determining the function of a nucleic acid (*e.g.,* coding function, ability to hybridize to another nucleic acid) are well-known in the art. Similarly, methods for determining protein function are well-known. For example, the DNA-binding function of a polypeptide can be determined, for example, by filter-binding, electrophoretic mobility-shift, or immunoprecipitation assays. DNA cleavage can be assayed by gel electrophoresis. See Ausubel *et al., supra.* The ability of a protein to interact with another protein can be determined, for example, by co-immunoprecipitation, two-hybrid assays or complementation, both genetic and biochemical. See, for example, Fields et al. (1989) Nature 340:245-246; U.S. Patent No. 5,585,245 and PCT WO 98/44350.

A "vector" is capable of transferring gene sequences to target cells. Typically, "vector construct," "expression vector," and "gene transfer vector," mean any nucleic acid construct capable of directing the expression of a gene of interest and which can transfer gene sequences to target cells. Thus, the term includes cloning, and expression vehicles, as well as integrating vectors.

A "reporter gene" or "reporter sequence" refers to any sequence that produces a protein product that is easily measured, preferably although not necessarily in a routine assay. Suitable reporter genes include, but are not limited to, sequences encoding proteins that mediate antibiotic resistance (*e.g*., ampicillin resistance, neomycin resistance, G418 resistance, puromycin resistance), sequences encoding colored or fluorescent or luminescent proteins (*e.g*., green fluorescent protein, enhanced green fluorescent protein, red fluorescent protein, luciferase), and proteins which mediate enhanced cell growth and/or gene amplification (*e.g*., dihydrofolate reductase). Epitope tags include, for example, one or more copies of FLAG, His, myc, Tap, HA or any detectable amino acid sequence. "Expression tags" include sequences that encode reporters that may be operably linked to a desired gene sequence in order to monitor expression of the gene of interest.

The terms "subject" and "patient" are used interchangeably and refer to mammals such as human patients and non-human primates, as well as experimental animals such as rabbits, dogs, cats, rats, mice, and other animals. Accordingly, the term "subject" or "patient" as used herein means any mammalian patient or subject to which the altered cells and/or proteins produced by the altered cells can be administered. Subjects of the present invention include those having an LSD (MPS I) and/or MPSII disorder.

### Nucleases

One or more nucleases, *i.e.* a pair of ZFNs, is used for targeted introduction of the IDUA or IDS transgene. The nucleases are non-naturally occurring, *i.e.,* engineered in the DNA-binding molecule (also referred to as a DNA-binding domain) and/or cleavage domain. Zinc finger nucleases may comprise heterologous DNA-binding and cleavage domains.

### A. DNA-binding domains

The DNA binding domain of one or more of the nucleases used for *in vivo* cleavage and/or targeted cleavage of the genome of a cell comprises a zinc finger protein. The zinc finger protein is non-naturally occurring in that it is engineered to bind to a target site of choice. *See,* for example, *See,* for example, Beerli et al. (2002) Nature Biotechnol.20:135-141; Pabo et al. (2001) Ann. Rev. Biochem.70:313-340; Isalan et al. (2001) Nature Biotechnol.19:656-660; Segal et al. (2001) Curr. Opin. Biotechnol. 12:632-637; Choo et al. (2000) Curr. Opin. Struct. Biol.10:411-416; U.S. Patent Nos. 6,453,242; 6,534,261; 6,599,692; 6,503,717; 6,689,558; 7,030,215; 6,794,136; 7,067,317; 7,262,054; 7,070,934; 7,361,635; 7,253,273; and U.S. Patent Publication Nos. 2005/0064474; 2007/0218528; 2005/0267061.

An engineered zinc finger binding domain can have a novel binding specificity, compared to a naturally-occurring zinc finger protein. Engineering methods include, but are not limited to, rational design and various types of selection. Rational design includes, for example, using databases comprising triplet (or quadruplet) nucleotide sequences and individual zinc finger amino acid sequences, in which each triplet or quadruplet nucleotide sequence is associated with one or more amino acid sequences of zinc fingers which bind the particular triplet or quadruplet sequence. See, for example, co-owned U.S. Patents 6,453,242 and 6,534,261.

Exemplary selection methods, including phage display and two-hybrid systems, are disclosed in US Patents 5,789,538; 5,925,523; 6,007,988; 6,013,453; 6,410,248; 6,140,466; 6,200,759; and 6,242,568; as well as WO 98/37186; WO 98/53057; WO 00/27878; and WO 01/88197. In addition, enhancement of binding specificity for zinc finger binding domains has been described, for example, in co-owned WO 02/077227.

In addition, as disclosed in these and other references, zinc finger domains and/or multi-fingered zinc finger proteins may be linked together using any suitable linker sequences, including for example, linkers of 5 or more amino acids in length. See, also, U.S. Patent Nos. 8,772,453; 6,479,626; 6,903,185; and 7,153,949 for exemplary linker sequences-. The proteins described herein may include any combination of suitable linkers between the individual zinc fingers of the protein.

Selection of target sites; ZFPs and methods for design and construction of fusion proteins (and polynucleotides encoding same) are known to those of skill in the art and described in detail in U.S. Patent Nos. 6,140,081; 5,789,538; 6,453,242; 6,534,261; 5,925,523; 6,007,988; 6,013,453; 6,200,759; WO 95/19431; WO 96/06166; WO 98/53057; WO 98/54311; WO 00/27878; WO 01/60970 WO 01/88197; WO 02/099084; WO 98/53058; WO 98/53059; WO 98/53060; WO 02/016536 and WO 03/016496.

In addition, as disclosed in these and other references, zinc finger domains and/or multi-fingered zinc finger proteins may be linked together using any suitable linker sequences, including for example, linkers of 5 or more amino acids in length. See, also, U.S. Patent Nos. 6,479,626; 6,903,185; and 7,153,949 for exemplary linker sequences 6 or more amino acids in length. The proteins described herein may include any combination of suitable linkers between the individual zinc fingers of the protein.

### B. Cleavage Domains

ZFP DNA-binding domains are fused to nuclease domains to create ZFNs - a functional entity that is able to recognize its intended nucleic acid target through its engineered (ZFP) DNA binding domain and cause the DNA to be cut near the ZFP binding site via the nuclease activity. See, *e.g.,* Kim et al. (1996) Proc Natl Acad Sci USA 93(3): 1156-1160. More recently, ZFNs have been used for genome modification in a variety of organisms. *See,* for example, United States Patent Publications 20030232410; 20050208489; 20050026157; 20050064474; 20060188987; 20060063231; and International Publication WO 07/014275.

The cleavage domain may be heterologous to the zinc finger DNA-binding domain, for example a cleavage domain from a nuclease. Heterologous cleavage domains can be obtained from any endonuclease or exonuclease. Exemplary endonucleases from which a cleavage domain can be derived include, but are not limited to, restriction endonucleases and homing endonucleases. *See,* for example, 2002-2003 Catalogue, New England Biolabs, Beverly, MA; and Belfort et al. (1997) Nucleic Acids Res.25:3379-3388*.* Additional enzymes which cleave DNA are known *(e.g.,* S1 Nuclease; mung bean nuclease; pancreatic DNase I; micrococcal nuclease; yeast HO endonuclease; *see* also Linn et al. (eds.) Nucleases, Cold Spring Harbor Laboratory Press, 1993). One or more of these enzymes (or functional fragments thereof) can be used as a source of cleavage domains and cleavage half-domains.

Similarly, a cleavage half-domain can be derived from any nuclease or portion thereof, as set forth above, that requires dimerization for cleavage activity. In general, two fusion proteins are required for cleavage if the fusion proteins comprise cleavage half-domains. Alternatively, a single protein comprising two cleavage half-domains can be used. The two cleavage half-domains can be derived from the same endonuclease (or functional fragments thereof), or each cleavage half-domain can be derived from a different endonuclease (or functional fragments thereof). In addition, the target sites for the two fusion proteins are preferably disposed, with respect to each other, such that binding of the two fusion proteins to their respective target sites places the cleavage half-domains in a spatial orientation to each other that allows the cleavage half-domains to form a functional cleavage domain, *e.g*., by dimerizing. Thus, in certain embodiments, the near edges of the target sites are separated by 5-8 nucleotides or by 15-18 nucleotides. However, any integral number of nucleotides or nucleotide pairs can intervene between two target sites (*e.g*., from 2 to 50 nucleotide pairs or more). In general, the site of cleavage lies between the target sites.

Restriction endonucleases (restriction enzymes) are present in many species and are capable of sequence-specific binding to DNA (at a recognition site), and cleaving DNA at or near the site of binding. Certain restriction enzymes (*e.g*., Type IIS) cleave DNA at sites removed from the recognition site and have separable binding and cleavage domains. For example, the Type IIS enzyme *Fok* I catalyzes double-stranded cleavage of DNA, at 9 nucleotides from its recognition site on one strand and 13 nucleotides from its recognition site on the other. *See,* for example, U.S. Patents 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31,978-31,982. Thus, in one embodiment, fusion proteins comprise the cleavage domain (or cleavage half-domain) from at least one Type IIS restriction enzyme and one or more zinc finger binding domains, which may or may not be engineered.

An exemplary Type IIS restriction enzyme, whose cleavage domain is separable from the binding domain, is *Fok* I. This particular enzyme is active as a dimer. Bitinaite et al. (1998) Proc. Natl. Acad. Sci. USA 95: 10,570-10,575. Accordingly, for the purposes of the present disclosure, the portion of the *Fok* I enzyme used in the disclosed fusion proteins is considered a cleavage half-domain. Thus, for targeted double-stranded cleavage and/or targeted replacement of cellular sequences using zinc finger-*Fok* I fusions, two fusion proteins, each comprising a *Fok*I cleavage half-domain, can be used to reconstitute a catalytically active cleavage domain. Alternatively, a single polypeptide molecule containing a zinc finger binding domain and two *Fok* I cleavage half-domains can also be used. Parameters for targeted cleavage and targeted sequence alteration using zinc finger*-Fok* I fusions are provided elsewhere in this disclosure.

A cleavage domain or cleavage half-domain can be any portion of a protein that retains cleavage activity, or that retains the ability to multimerize (*e.g*., dimerize) to form a functional cleavage domain.

Exemplary Type IIS restriction enzymes are described in U.S. Patent 7,888,121. Additional restriction enzymes also contain separable binding and cleavage domains, and these are contemplated by the present disclosure. *See,* for example, Roberts et al. (2003) Nucleic Acids Res.31:418-420.

In certain embodiments, the cleavage domain comprises one or more engineered cleavage half-domain (also referred to as dimerization domain mutants) that minimize or prevent homodimerization, as described, for example, in U.S. Patent Nos. 8,772,453; 8,623,618; 8,409,861; 8,034,598; 7,914,796; and 7,888,121. Amino acid residues at positions 446, 447, 479, 483, 484, 486, 487, 490, 491, 496, 498, 499, 500, 531, 534, 537, and 538 of *Fok*I are all targets for influencing dimerization of the *Fok*I cleavage half-domains.

Exemplary engineered cleavage half-domains of *Fok*I that form obligate heterodimers include a pair in which a first cleavage half-domain includes mutations at amino acid residues at positions 490 and 538 of *Fok*I and a second cleavage half-domain includes mutations at amino acid residues 486 and 499.

Thus, in one embodiment, a mutation at 490 replaces Glu (E) with Lys (K); the mutation at 538 replaces Iso (I) with Lys (K); the mutation at 486 replaced Gln (Q) with Glu (E); and the mutation at position 499 replaces Iso (I) with Lys (K). Specifically, the engineered cleavage half-domains described herein were prepared by mutating positions 490 (E→K) and 538 (I→K) in one cleavage half-domain to produce an engineered cleavage half-domain designated "E490K:I538K" and by mutating positions 486 (Q→E) and 499 (I→L) in another cleavage half-domain to produce an engineered cleavage half-domain designated "Q486E:I499L". The engineered cleavage half-domains described herein are obligate heterodimer mutants in which aberrant cleavage is minimized or abolished. U.S. Patent Nos. 7,914,796 and 8,034,598. In certain embodiments, the engineered cleavage half-domain comprises mutations at positions 486, 499 and 496 (numbered relative to wild-type FokI), for instance mutations that replace the wild type Gln (Q) residue at position 486 with a Glu(E) residue, the wild type Iso (I) residue at position 499 with a Leu (L) residue and the wild-type Asn (N) residue at position 496 with an Asp (D) or Glu (E) residue (also referred to as a "ELD" and "ELE" domains, respectively). In other embodiments, the engineered cleavage half-domain comprises mutations at positions 490, 538 and 537 (numbered relative to wild-type FokI), for instance mutations that replace the wild type Glu (E) residue at position 490 with a Lys (K) residue, the wild type Iso (I) residue at position 538 with a Lys (K) residue, and the wild-type His (H) residue at position 537 with a Lys (K) residue or a Arg (R) residue (also referred to as "KKK" and "KKR" domains, respectively). In other embodiments, the engineered cleavage half-domain comprises mutations at positions 490 and 537 (numbered relative to wild-type FokI), for instance mutations that replace the wild type Glu (E) residue at position 490 with a Lys (K) residue and the wild-type His (H) residue at position 537 with a Lys (K) residue or a Arg (R) residue (also referred to as "KIK" and "KIR" domains, respectively). *See, e.g.,* U.S. Patent No. 8,772,453. In other embodiments, the engineered cleavage half domain comprises the "Sharkey" and/or "Sharkey" mutations (see Guo et al, (2010) J. Mol. Biol. 400(1):96-107).

Engineered cleavage half-domains described herein can be prepared using any suitable method, for example, by site-directed mutagenesis of wild-type cleavage half-domains (*Fok* I) as described in U.S. Patent Nos. 7,888,121; 7,914,796; 8,034,598; and 8,623,618.

Alternatively, nucleases may be assembled *in vivo* at the nucleic acid target site using so-called "split-enzyme" technology (*see, e.g.* U.S. Patent Publication No. 20090068164). Components of such split enzymes may be expressed either on separate expression constructs, or can be linked in one open reading frame where the individual components are separated, for example, by a self-cleaving 2A peptide or IRES sequence. Components may be individual zinc finger binding domains or domains of a meganuclease nucleic acid binding domain.

Nucleases can be screened for activity prior to use, for example in a yeast-based chromosomal system as described in U.S. Patent No. 8,563,314. Expression of the nuclease may be under the control of a constitutive promoter or an inducible promoter, for example the galactokinase promoter which is activated (de-repressed) in the presence of raffinose and/or galactose and repressed in presence of glucose.

The nuclease(s) as described herein may make one or more double-stranded and/or single-stranded cuts in the target site. In certain embodiments, the nuclease comprises a catalytically inactive cleavage domain (*e.g.,* FokI). *See, e.g.,* U.S. Patent No. 9,200,266; 8,703,489 and Guillinger et al. (2014) Nature Biotech. 32(6):577-582. The catalytically inactive cleavage domain may, in combination with a catalytically active domain act as a nickase to make a single-stranded cut. Therefore, two nickases can be used in combination to make a double-stranded cut in a specific region. Additional nickases are also known in the art, for example, McCaffery et al. (2016) Nucleic Acids Res. 44(2):e11. doi: 10.1093/nar/gkv878. Epub 2015 Oct 19.

### Target Sites

As described in detail above, DNA domains can be engineered to bind to any sequence of choice in a locus, for example an albumin or other safe-harbor gene. An engineered DNA-binding domain can have a novel binding specificity, compared to a naturally-occurring DNA-binding domain. Engineering methods include, but are not limited to, rational design and various types of selection. Rational design includes, for example, using databases comprising triplet (or quadruplet) nucleotide sequences and individual (*e.g*., zinc finger) amino acid sequences, in which each triplet or quadruplet nucleotide sequence is associated with one or more amino acid sequences of DNA binding domain which bind the particular triplet or quadruplet sequence. *See,* for example, co-owned U.S. Patents 6,453,242 and 6,534,261. Rational design of TAL-effector domains can also be performed. *See, e.g.,* U.S. Publication No. 20110301073.

Exemplary selection methods applicable to DNA-binding domains, including phage display and two-hybrid systems, are disclosed in US Patents 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237.

Selection of target sites; nucleases and methods for design and construction of fusion proteins (and polynucleotides encoding same) are known to those of skill in the art and described in detail in U.S. Patent Application Publication Nos. 20050064474 and 20060188987.

In addition, as disclosed in these and other references, DNA-binding domains (*e.g*., multi-fingered zinc finger proteins) may be linked together using any suitable linker sequences, including for example, linkers of 5 or more amino acids. *See, e.g.,* U.S. Patent Nos. 6,479,626; 6,903,185; and 7,153,949 for exemplary linker sequences 6 or more amino acids in length. The proteins described herein may include any combination of suitable linkers between the individual DNA-binding domains of the protein. *See, also,* U.S. Patent No. 8,586,526.

### Donors

As noted above, insertion of an exogenous sequence (also called a "donor sequence" or "donor"), for example for correction of a mutant gene or for increased expression of a gene encoding a protein lacking or deficient in MPS I and/or MPS II disease (IDUA or IDS) is provided. It will be readily apparent that the donor sequence is typically not identical to the genomic sequence where it is placed. A donor sequence can contain a non-homologous sequence flanked by two regions of homology to allow for efficient HDR at the location of interest. Additionally, donor sequences can comprise a vector molecule containing sequences that are not homologous to the region of interest in cellular chromatin. A donor molecule can contain several, discontinuous regions of homology to cellular chromatin. For example, for targeted insertion of sequences not normally present in a region of interest, said sequences can be present in a donor nucleic acid molecule and flanked by regions of homology to sequence in the region of interest.

Described herein are methods of targeted insertion of a transgene encoding an IDUA or IDS protein for insertion into a chosen location. Polynucleotides for insertion can also be referred to as "exogenous" polynucleotides, "donor" polynucleotides or molecules or "transgenes." The donor polynucleotide can be DNA or RNA, single-stranded and/or double-stranded and can be introduced into a cell in linear or circular form. *See, e.g.,* U.S. Patent Nos. 8,703,489 and 9,005,973. The donor sequence(s) can also be contained within a DNA MC, which may be introduced into the cell in circular or linear form. *See, e.g.,* U.S. Patent Publication No. 20140335063. If introduced in linear form, the ends of the donor sequence can be protected (*e.g*., from exonucleolytic degradation) by methods known to those of skill in the art. For example, one or more dideoxynucleotide residues are added to the 3' terminus of a linear molecule and/or self-complementary oligonucleotides are ligated to one or both ends. *See,* for example, Chang et al. (1987) Proc. Natl. Acad. Sci. USA 84:4959-4963; Nehls et al. (1996) Science 272:886-889. Additional methods for protecting exogenous polynucleotides from degradation include, but are not limited to, addition of terminal amino group(s) and the use of modified internucleotide linkages such as, for example, phosphorothioates, phosphoramidates, and O-methyl ribose or deoxyribose residues.

A polynucleotide can be introduced into a cell as part of a vector molecule having additional sequences such as, for example, replication origins, and genes encoding antibiotic resistance. Moreover, donor polynucleotides can be introduced as naked nucleic acid, as nucleic acid complexed with an agent such as a liposome or poloxamer, or can be delivered by viruses (*e.g*., adenovirus, AAV, herpesvirus, retrovirus, lentivirus and integrase defective lentivirus (IDLV)).

The donor is generally inserted so that its expression is driven by the endogenous promoter at the integration site, namely the promoter that drives expression of the endogenous gene into which the donor is inserted (albumin).

The transgene may be inserted into an albumin locus such that some (N-terminal and/or C-terminal to the transgene encoding the lysosomal enzyme) or none of the endogenous albumin sequences are expressed, for example as a fusion with the transgene encoding the IDUA or IDS protein(s).

When endogenous sequences (endogenous or part of the transgene) are expressed with the transgene, the endogenous sequences (albumin) may be full-length sequences (wild-type or mutant) or partial sequences. Preferably the endogenous sequences are functional. Non-limiting examples of the function of these full length or partial sequences (albumin) include increasing the serum half-life of the polypeptide expressed by the transgene (*e.g*., therapeutic gene) and/or acting as a carrier.

Furthermore, although not required for expression, exogenous sequences may also include transcriptional or translational regulatory sequences, for example, enhancers, insulators, internal ribosome entry sites, sequences encoding 2A peptides and/or polyadenylation signals.

In some cases, the donor may be an endogenous gene (IDUA or IDS) that has been modified. For instance, codon optimization may be performed on the endogenous gene to produce a donor. Furthermore, although antibody response to enzyme replacement therapy varies with respect to the specific therapeutic enzyme in question and with the individual patient, a significant immune response has been seen in many MPS I or MPS II disease patients being treated with enzyme replacement with wild-type IDUA or IDS. In addition, the relevance of these antibodies to the efficacy of treatment is also variable (see Katherine Ponder, (2008) J Clin Invest 118(8):2686). Thus, the compositions of the current invention can comprise the generation of donor with modified sequences as compared to wild-type IDUA or IDS, including, but not limited to, modifications that produce functionally silent amino acid changes at sites known to be priming epitopes for endogenous immune responses, and/or truncations such that the polypeptide produced by such a donor is less immunogenic.

MPS I and MPS II disease patients often have neurological sequelae due the lack of the missing IDUA or IDS enzyme in the brain. Unfortunately, it is often difficult to deliver therapeutics to the brain via the blood due to the impermeability of the blood brain barrier. Thus, the compositions of the invention may be used in conjunction with methods to increase the delivery of the therapeutic into the brain, including but not limited to methods that cause a transient opening of the tight junctions between cells of the brain capillaries such as transient osmotic disruption through the use of an intracarotid administration of a hypertonic mannitol solution, the use of focused ultrasound and the administration of a bradykinin analogue. Alternatively, therapeutics can be designed to utilize receptors or transport mechanisms for specific transport into the brain. Examples of specific receptors that may be used include the transferrin receptor, the insulin receptor or the low-density lipoprotein receptor related proteins 1 and 2 (LRP-1 and LRP-2). LRP is known to interact with a range of secreted proteins such as apoE, tPA, PAI-1 etc, and so fusing a recognition sequence from one of these proteins for LRP may facilitate transport of the enzyme into the brain, following expression in the liver of the therapeutic protein and secretion into the blood stream (see Gabathuler, (2010) *ibid*)*.*

### Cells

The IDUA and/or IDS transgene is integrated in a targeted manner into the liver cell's genome using a pair of zinc finger nucleases (ZFNs) targeted to an endogenous albumin gene. Unlike random integration, nuclease-mediated targeted integration ensures that the transgene is integrated into a specified gene. The transgene may be integrated anywhere in the target gene. In certain embodiments, the transgene is integrated at or near the nuclease binding and/or cleavage site, for example, within 1-300 (or any number of base pairs therebetween) base pairs upstream or downstream of the site of cleavage and/or binding site, more preferably within 1-100 base pairs (or any number of base pairs therebetween) of either side of the cleavage and/or binding site, even more preferably within 1 to 50 base pairs (or any number of base pairs therebetween) of either side of the cleavage and/or binding site. In certain embodiments, the integrated sequence does not include any vector sequences (*e.g*., viral vector sequences).

### Delivery

The nucleases, polynucleotides encoding these nucleases, donor polynucleotides and compositions comprising the proteins and/or polynucleotides described herein may be delivered *in vivo* or *ex vivo* by any suitable means.

Methods of delivering nucleases as described herein are described, for example, in U.S. Patent Nos. 6,453,242; 6,503,717; 6,534,261; 6,599,692; 6,607,882; 6,689,558; 6,824,978; 6,933,113; 6,979,539; 7,013,219; and 7,163,824.

Any vector systems may be used including, but not limited to, plasmid vectors, retroviral vectors, lentiviral vectors, adenovirus vectors, poxvirus vectors; herpesvirus vectors and adeno-associated virus vectors, etc. *See, also,* U.S. Patent Nos. 6,534,261; 6,607,882; 6,824,978; 6,933,113; 6,979,539; 7,013,219; and 7,163,824.

Conventional viral and non-viral based gene transfer methods can be used to introduce nucleic acids encoding nucleases and donor constructs in cells (*e.g.,* mammalian cells) and target tissues. Non-viral vector delivery systems include DNA plasmids, naked nucleic acid, and nucleic acid complexed with a delivery vehicle such as a liposome or poloxamer. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds.) (1995); and Yu et al., Gene Therapy 1:13-26 (1994).

Methods of non-viral delivery of nucleic acids include electroporation, lipofection, microinjection, biolistics, virosomes, liposomes, immunoliposomes, polycation or lipid:nucleic acid conjugates, naked DNA, artificial virions, and agent-enhanced uptake of DNA. Sonoporation using, *e.g.,* the Sonitron 2000 system (Rich-Mar) can also be used for delivery of nucleic acids.

Additional exemplary nucleic acid delivery systems include those provided by Amaxa Biosystems (Cologne, Germany), Maxcyte, Inc. (Rockville, Maryland), BTX Molecular Delivery Systems (Holliston, MA) and Copernicus Therapeutics Inc, (*see* for example US6008336). Lipofection is described in *e.g.,* U.S. Patent Nos. 5,049,386; 4,946,787; and 4,897,355) and lipofection reagents are sold commercially (*e.g.*, Transfectam™ and Lipofectin™). Cationic and neutral lipids that are suitable for efficient receptor-recognition lipofection of polynucleotides include those of Felgner, WO 91/17424, WO 91/16024.

The preparation of lipid:nucleic acid complexes, including targeted liposomes such as immunolipid complexes, is well known to one of skill in the art (*see, e.g.,* Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

Additional methods of delivery include the use of packaging the nucleic acids to be delivered into EnGeneIC delivery vehicles (EDVs). These EDVs are specifically delivered to target tissues using bispecific antibodies where one arm of the antibody has specificity for the target tissue and the other has specificity for the EDV. The antibody brings the EDVs to the target cell surface and then the EDV is brought into the cell by endocytosis. Once in the cell, the contents are released (*see* MacDiarmid et al (2009) Nature Biotechnology 27(7):643).

The use of RNA or DNA viral based systems for the delivery of nucleic acids encoding engineered ZFPs take advantage of highly evolved processes for targeting a virus to specific cells in the body and trafficking the viral payload to the nucleus. Viral vectors can be administered directly to subjects (*in vivo*) or they can be used to treat cells *in vitro* and the modified cells are administered to subjects (*ex vivo*)*.* Conventional viral based systems for the delivery of ZFPs include, but are not limited to, retroviral, lentivirus, adenoviral, adeno-associated, vaccinia and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system depends on the target tissue. Retroviral vectors are comprised of *cis*-acting long terminal repeats with packaging capacity for up to 6-10 kb of foreign sequence. The minimum *cis*-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immunodeficiency virus (SIV), human immunodeficiency virus (HIV), and combinations thereof (*see, e.g.,* Buchscher et al., J. Virol. 66:2731-2739 (1992); Johann et al., J. Virol. 66:1635-1640 (1992); Sommerfelt et al., Virol. 176:58-59 (1990); Wilson et al., J. Virol. 63:2374-2378 (1989); Miller et al., J. Virol. 65:2220-2224 (1991); PCT/US94/05700).

In applications in which transient expression is preferred, adenoviral based systems can be used. Adenoviral based vectors are capable of very high transduction efficiency in many cell types and do not require cell division. With such vectors, high titer and high levels of expression have been obtained. This vector can be produced in large quantities in a relatively simple system. Adeno-associated virus ("AAV") vectors are also used to transduce cells with target nucleic acids, *e.g.,* in the *in vitro* production of nucleic acids and peptides, and for *in vivo* and *ex vivo* gene therapy procedures (*see, e.g.,* West et al., Virology 160:38-47 (1987); U.S. Patent No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

At least six viral vector approaches are currently available for gene transfer in clinical trials, which utilize approaches that involve complementation of defective vectors by genes inserted into helper cell lines to generate the transducing agent.

pLASN and MFG-S are examples of retroviral vectors that have been used in clinical trials (Dunbar et al., Blood 85:3048-305 (1995); Kohn et al., Nat. Med. 1:1017-102 (1995); Malech et al., PNAS 94:22 12133-12138 (1997)). PA317/pLASN was the first therapeutic vector used in a gene therapy trial. (Blaese et al., Science 270:475-480 (1995)). Transduction efficiencies of 50% or greater have been observed for MFG-S packaged vectors. (Ellem et al., Immunol Immunother. 44(1):10-20 (1997); Dranoff et al., Hum. Gene Ther. 1:111-2 (1997).

Recombinant adeno-associated virus vectors (rAAV) are a promising alternative gene delivery systems based on the defective and nonpathogenic parvovirus adeno-associated type 2 virus. All vectors are derived from a plasmid that retains only the AAV 145 bp inverted terminal repeats flanking the transgene expression cassette. Efficient gene transfer and stable transgene delivery due to integration into the genomes of the transduced cell are key features for this vector system. (Wagner et al., Lancet 351:9117 1702-3 (1998), Kearns et al., Gene Ther. 9:748-55 (1996)). Other AAV serotypes, including by non-limiting example, AAV1, AAV3, AAV4, AAV5, AAV6, AAV8, AAV 8.2, AAV9 and AAV rh10 and pseudotyped AAV such as AAV2/8, AAV2/5 and AAV2/6 can also be used in accordance with the present invention.

Replication-deficient recombinant adenoviral vectors (Ad) can be produced at high titer and readily infect a number of different cell types. Most adenovirus vectors are engineered such that a transgene replaces the Ad E1a, E1b, and/or E3 genes; subsequently the replication defective vector is propagated in human 293 cells that supply deleted gene function in *trans.* Ad vectors can transduce multiple types of tissues *in vivo,* including non-dividing, differentiated cells such as those found in liver, kidney and muscle. Conventional Ad vectors have a large carrying capacity. An example of the use of an Ad vector in a clinical trial involved polynucleotide therapy for anti-tumor immunization with intramuscular injection (Sterman et al., Hum. Gene Ther. 7:1083-9 (1998)). Additional examples of the use of adenovirus vectors for gene transfer in clinical trials include Rosenecker et al., Infection 24:1 5-10 (1996); Sterman et al., Hum. Gene Ther. 9:7 1083-1089 (1998); Welsh et al., Hum. Gene Ther. 2:205-18 (1995); Alvarez et al., Hum. Gene Ther. 5:597-613 (1997); Topf et al., Gene Ther. 5:507-513 (1998); Sterman et al., Hum. Gene Ther. 7:1083-1089 (1998).

Packaging cells are used to form virus particles that are capable of infecting a host cell. Such cells include 293 cells, which package adenovirus, and ψ2 cells or PA317 cells, which package retrovirus. Viral vectors used in gene therapy are usually generated by a producer cell line that packages a nucleic acid vector into a viral particle. The vectors typically contain the minimal viral sequences required for packaging and subsequent integration into a host (if applicable), other viral sequences being replaced by an expression cassette encoding the protein to be expressed. The missing viral functions are supplied in *trans* by the packaging cell line. For example, AAV vectors used in gene therapy typically only possess inverted terminal repeat (ITR) sequences from the AAV genome which are required for packaging and integration into the host genome. Viral DNA is packaged in a cell line, which contains a helper plasmid encoding the other AAV genes, namely *rep* and *cap,* but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, *e.g.,* heat treatment to which adenovirus is more sensitive than AAV.

In many gene therapy applications, it is desirable that the gene therapy vector be delivered with a high degree of specificity to a particular tissue type. Accordingly, a viral vector can be modified to have specificity for a given cell type by expressing a ligand as a fusion protein with a viral coat protein on the outer surface of the virus. The ligand is chosen to have affinity for a receptor known to be present on the cell type of interest. For example, Han et al., Proc. Natl. Acad. Sci. USA 92:9747-9751 (1995), reported that Moloney murine leukemia virus can be modified to express human heregulin fused to gp70, and the recombinant virus infects certain human breast cancer cells expressing human epidermal growth factor receptor. This principle can be extended to other virus-target cell pairs, in which the target cell expresses a receptor and the virus expresses a fusion protein comprising a ligand for the cell-surface receptor. For example, filamentous phage can be engineered to display antibody fragments (*e.g*., FAB or Fv) having specific binding affinity for virtually any chosen cellular receptor. Although the above description applies primarily to viral vectors, the same principles can be applied to nonviral vectors. Such vectors can be engineered to contain specific uptake sequences which favor uptake by specific target cells.

Gene therapy vectors can be delivered *in vivo* by administration to an individual patient, typically by systemic administration (*e.g*., intravenous, intraperitoneal, intramuscular, subdermal, or intracranial infusion) or topical application, as described below. Alternatively, vectors can be delivered to cells *ex vivo,* such as cells explanted from an individual patient (*e.g.,* lymphocytes, bone marrow aspirates, tissue biopsy) or universal donor hematopoietic stem cells, followed by reimplantation of the cells into a patient, usually after selection for cells which have incorporated the vector.

Vectors (*e.g*., retroviruses, adenoviruses, liposomes, etc.) containing nucleases and/or donor constructs can also be administered directly to an organism for transduction of cells *in vivo.* Alternatively, naked DNA can be administered. Administration is by any of the routes normally used for introducing a molecule into ultimate contact with blood or tissue cells including, but not limited to, injection, infusion, topical application and electroporation. Suitable methods of administering such nucleic acids are available and well known to those of skill in the art, and, although more than one route can be used to administer a particular composition, a particular route can often provide a more immediate and more effective reaction than another route.

Vectors suitable for introduction of polynucleotides described herein include non-integrating lentivirus vectors (IDLV). *See,* for example, Ory et al. (1996) Proc. Natl. Acad. Sci. USA 93:11382-11388; Dull et al. (1998) J. Virol. 72:8463-8471; Zuffery et al. (1998) J. Virol. 72:9873-9880; Follenzi et al. (2000) Nature Genetics 25:217-222; U.S. Patent Publication No 2009/054985.

Pharmaceutically acceptable carriers are determined in part by the particular composition being administered, as well as by the particular method used to administer the composition. Accordingly, there is a wide variety of suitable formulations of pharmaceutical compositions available, as described below (*see, e.g.,* Remington's Pharmaceutical Sciences, 17th ed., 1989).

It will be apparent that the nuclease-encoding sequences and donor constructs can be delivered using the same or different systems. For example, a donor polynucleotide can be carried by a plasmid, while the one or more nucleases can be carried by an AAV vector. Furthermore, the different vectors can be administered by the same or different routes (intramuscular injection, tail vein injection, other intravenous injection, intraperitoneal administration and/or intramuscular injection. The vectors can be delivered simultaneously or in any sequential order.

Formulations for both *ex vivo* and *in vivo* administrations include suspensions in liquid or emulsified liquids. The active ingredients often are mixed with excipients which are pharmaceutically acceptable and compatible with the active ingredient. Suitable excipients include, for example, water, saline, dextrose, glycerol, ethanol or the like, and combinations thereof. In addition, the composition may contain minor amounts of auxiliary substances, such as, wetting or emulsifying agents, pH buffering agents, stabilizing agents or other reagents that enhance the effectiveness of the pharmaceutical composition.

### Applications

The invention relates to the treatment and/or prevention of MPS I disease (*e.g.* a lysosomal storage disease) and/or an MPS I disease. Treatment comprises insertion of one or more corrective disease-associated genes (IDUA or IDS) into an albumin locus in a liver cell for expression of the needed enzyme(s) and release into the blood stream. The transgene may encode a protein comprising a codon optimized IDUA or IDS transgene; and/or a transgene in which epitopes may be removed without functionally altering the protein.

The invention is particularly useful to correct or restore functionality in subjects with MPS I disease.

By way of non-limiting examples, production of the defective or missing proteins accomplished and used to treat MPS I and/or MPS II disease. Nucleic acid donors encoding the missing protein is inserted into a safe harbor locus (albumin) and expressed using the promoter present at the safe harbor.

In some applications, an endogenous gene may be knocked out by use of the compositions of the invention, e.g. knocking out an aberrant gene regulator or an aberrant disease associated gene. In some applications, an aberrant endogenous gene may be replaced, either functionally or *in situ,* with a wild type version of the gene. The inserted gene may also be altered to improve the expression of the therapeutic IDUA or IDS protein or to reduce its immunogenicity. In some applications, the inserted IDUA or IDS encoding transgene is a fusion protein to increase its transport into a selected tissue such as the brain.

The following Examples relate to exemplary embodiments of the present disclosure.

### EXAMPLES

### Example 1: Design and construction of mouse ZFN reagents and IDUA donor template

As the target sequences for the clinical candidate ZFNs and the human albumin donor homology arms are not conserved in the mouse albumin locus, mouse surrogate reagents were developed for use in these mouse studies. The mouse surrogate reagents included a pair of ZFN rAAV vectors (SB-48641, SB-31523, shown below in Table I and Table II, see U.S. Patent Application 14/872,537) targeting a homologous site in intron 1 of the mouse albumin gene as well as a donor that encodes a promoterless human IDUA transgene (hIDUA) flanked by arms with homology to the mouse locus (SB-mu-IDUA). The ratio of ZFN:ZFN:Donor used in this study was 1:1:8. For this mouse study, the surrogate reagents were packaged and delivered using serotype rAAV2/8, as it confers superior transduction and faster transgene expression than rAAV2/6 vectors in mice *in vivo.* The rAAV2/8 vectors were diluted into the formulation buffer, phosphate-buffered saline (PBS) supplemented with 35 mM NaCl and 5% glycerol, pH 7.1.

**Table 1: Mouse Albumin Designs**

| SBS # | Design | | | | |
|---|---|---|---|---|---|
| | F1 | F2 | F3 | F4 | F5 |
| **31523** | RSDNLSE (SEQ ID NO:1) | QSGNLAR (SEQ ID NO:2) | DRSNLSR (SEQ ID NO:3) | WRSSLRA (SEQ ID NO:4) | DSSDRKK (SEQ ID NO:5) |
| **48641** | TSGSLTR (SEQ ID NO:6) | RSDALST (SEQ ID NO:(7) | QSATRTK (SEQ ID NO:8) | LRHHLTR (SEQ ID NO:9) | QAGQRRV (SEQ ID NO:10) |

**Table 2: Target Sites of mouse albumin-specific zinc fingers**

| SBS # | Target site |
|---|---|
| **31523** | ttTCCTGTAACGATCGGgaactggcatc (SEQ ID NO:11) |
| **48641** | ctGAAGGTgGCAATGGTTcctctctgct (SEQ ID NO:12) |

The mouse albumin-specific ZFN pair was fully active.

### Example 2: Treatment of MPS I (IDUA knockout or Idua -/-) in a mouse model

### A. Materials and Methods

The MPS I (IDUA knockout or *Idua* -/-) mouse model used in this study was generated by Dr. Elizabeth Neufeld (UCLA, Los Angeles, CA) via disruption of the IDUA gene, in which a neomycin resistance cassette was inserted into exon 6 (Ohmi et al. (2003). Proc. Natl. Acad. Sci. USA 100(4):1902 -1907). Mice were then bred onto a C57BL/6 genetic background, and heterozygotes were selected to produce *Idua* -/- mice. This homozygous mutation leads to significant morphological, biochemical and neurological changes over the course of the animal's lifespan, and has proven to be a suitable model for the study of lysosomal pathophysiology. In particular, these MPS I mice have been shown to exhibit phenotypic features typical for MPS I, such as craniofacial abnormalities, neurological deficits, and increased GAG levels in tissues and urine (Hartung et al. (2004). Mol Ther. 9(6):866-75; Ou et al. (2014). Mol Genet Metab. 111(2):116-22). The *Idua-*/*-* mice begin to develop these symptoms after about 3-5 weeks and by 12-16 weeks of age extensive lysosomal accumulation of GAG is evident in the liver and central nervous system (CNS, *i.e.* brain, spinal cord and meninges). In addition, these MPS I mice show cognitive deficits/impairments (Hartung *et al. ibid;* Ou *et al. ibid*)*.*

Cohorts of mice were randomized and dosed between 4-10 weeks of age as litters became available. At least 2 mice per group were dosed on the same day.

Animals were administered cyclophosphamide to suppress a possible immunogenicity response to the hIDUA protein. All mice received a 200 µL intraperitoneal (IP) injection of cyclophosphamide (120 mg/kg) on the day before dosing and weekly thereafter through Week 12. After Week 12, the mice received 120 mg/kg once every two weeks until necropsy. The reason for the change in frequency of cyclophosphamide administration after Week 12 was due to observations in a few mice of mild alopecia near the injection site and acute mild bradykinesia, which are known side effects of cyclophosphamide. Previous studies have demonstrated efficient immunosuppression to attenuate loss of human IDUA activity in MPS I mice using a bimonthly injection schedule (Aronovich et al. (2007). J. Gene Med. 9:403-15), or as needed (see Ou et al (2014) Mol Genet Metabol 111(2):116).

Animals were randomized based on age and body weight for each gender. A single dose of test article or vehicle was administered on Day 1. Three mice from each group were euthanized 1 month after AAV injection. The remaining 5 mice from each group were euthanized 4 months after AAV injection. The total rAAV2/8 dose level for the ZFNs+Donor groups was l.5e l 2 vg/mouse, and for the Donor Only group was 1 .2e 12 vg/mouse. Groups and doses received are shown below in Table 3.

**Table 3: Group Designations and Dose Levels**

| **Group** | **Group Designation** | **Genotype** | **No. of Animals** | | **Each ZFN Dose Level (vg/mouse)** | **Donor Dose Level (vg/mouse)** | **Total AAV Dose (vg/mouse)** | **Total AAV Dose (vg/kg)¹** |
|---|---|---|---|---|---|---|---|---|
| | | | **Male** | **Female** | | | | |
| 1 | Formulation buffer control | C57BL/6 | 8 | 0 | 0 | 0 | 0 | 0 |
| 2 | Formulation buffer control | MPS I | 8 | 0 | 0 | 0 | 0 | 0 |
| 3 | Formulation **buffer** control | MPS I | 0 | 8 | 0 | 0 | 0 | 0 |
| 4 | ZFNs+Donor | MPS I | 8 | 0 | 1.5e11 | 1.2e12 | 1.5e12 | 7.5e13 |
| 5 | ZFNs+Donor | MPS I | 0 | 8 | 1.5e11 | 1.2e12 | 1.5e12 | 7.5e13 |
| 6 | Donor Only | MPS I | 4 | 4 | 0 | 1.2e12 | 1.2e12 | 6e13 |

**Collections and Measurements:** Blood samples for hematology and clinical chemistry analysis were collected from all animals prior to necropsy (Day 120) via submandibular bleed. Animals were fasted for at least 8 hours prior to serum chemistry collections. Blood samples for hematology analysis (target volume 100-150 µL) were collected in tubes containing potassium (K3) EDTA as an anticoagulant. Blood samples for clinical chemistry analysis (target volume 100-150 µL) were collected in tubes without anticoagulant and processed to serum.

For assessment of IDUA activity, blood samples (target volume 200 µL) were collected via submandibular bleed from all mice on Days 7, 14, 28, 60, 90, 104 and 120 into tubes containing heparin as an anticoagulant and processed to plasma.

For assessment of urinary glycosaminoglycan (GAG) levels, urine samples (approximately 10-100 µL) were collected from all mice by gently applying pressure to the urinary bladder on Days 7, 14, 21, 28, 42, 60, 74, 90, 104 and 120.

Genomic DNA Isolation from Mouse Tissue: Approximately 5 mg of frozen tissue was placed into a Lysis Matrix D tube (MP Biomedicals, Burlingame, CA) and 400 µL of Tissue and Cell Lysis Solution was added (Epicentre, Madison, WI). Homogenization was performed in a FastPrep®-24 Instrument (MP Biomedicals) by three rounds, 40 seconds/pulse of 4 m/s. The crude lysates were briefly centrifuged and then incubated for 30 minutes at 65°C followed by a brief centrifugation to clarify the lysate. The partially clarified lysate was placed on ice for 5 minutes and transferred to microcentrifuge tubes, followed by the addition of 400 µL of MPC Protein Precipitation Reagent (Epicentre). The mixture was briefly vortexed, and then centrifuged at 14,000 rpm for 10 minutes at 4°C. Following centrifugation, the clarified lysate was transferred to a new microcentrifuge tube and 1 mL of ice-cold isopropanol was added, mixed by inverting 30-50 times, and incubated at -20°C for 20 minutes. The mixture was centrifuged at 14,000 rpm for 10 minutes at 4°C and the supernatant removed. The resultant pellet was washed twice with 75% Ethanol and allowed to air dry. The DNA pellet was re-suspended in 100 µL TE Buffer and the DNA concentration (ng/µL) was determined using a NanoDrop spectrophotometer (Thermo Fisher Scientific, Waltham, MA).

Gene Modification Analysis: MiSeq Deep Sequencing: To evaluate the activity of mouse surrogate ZFN constructs in targeted hepatocytes, genomic DNA was isolated from the liver of each mouse. The ZFN target site was subjected to sequence analysis using the MiSeq system (Illumina, San Diego CA). A pair of oligonucleotide primers was designed for amplification of a 169-base pair (bp) fragment spanning the ZFN target site in the mouse albumin locus (Table 4) and to introduce binding sequences for a second round of amplification (in bold). The products of this polymerase chain reaction (PCR) amplification were purified and subjected to a second round of PCR with oligonucleotides designed to introduce an amplicon-specific identifier sequence ("barcode"), as well as terminal regions designed for binding sequencing oligonucleotide primers. The mixed population of bar-coded amplicons was then subjected to MiSeq analysis, a solid-phase sequencing procedure that allows the parallel analysis of thousands of samples on a single assay chip. The MiSeq protocol sequentially performs both forward and reverse sequencing reactions for each input oligonucleotide. Paired sequences are aligned and merged and were compared with the wild type sequence to map insertions and/or deletions ("indels"). ZFN activity is reported as "% indels", the fraction of sequenced amplicons that differ from wild type due to insertions or deletions.

**Table 4. Oligonucleotides Used for MiSeq Insertion/Deletion (indel) Analysis**

| **PCR#1: Albumin (intron 1) Specific Primers** | |
|---|---|
| Forward | **ACACGACGCTCTTCCGATCT**NNNNAAATCTTGAGTTTGAATGCACAGAT (SEQ ID NO:13) |
| Reverse | **GACGTGTGCTCTTCCGATCT**TTCACTGACCTAAGCTACTCCC (SEQ ID NO:14) |

hIDUA Western Blot: To examine expression of hIDUA in mouse hepatocytes, Western blots were performed on liver protein extracts. Approximately 50 mg of frozen tissue was placed into a Lysis Matrix D tube (MP Biomedicals, Burlingame, CA) and 500 µL of RIPA buffer supplemented with HALT Protease Inhibitor (Thermo Fisher Scientific) was added. Homogenization was performed in a FastPrep®-24 Instrument (MP Biomedicals) for five rounds, 45 seconds/pulse of 4.5 m/s. Tubes were placed on ice between rounds of homogenization. The crude lysates were then centrifuged at 14,000 rpm for 10 minutes at 4°C. Following centrifugation, the clarified lysate was transferred to a pre-chilled microcentrifuge tube. Protein concentration was then determined via the Pierce™ bicinchoninic acid (BCA) Protein Assay Kit (Thermo Fisher Scientific). Under denaturing conditions, 35 µg of total protein was separated on 4-12% NuPAGE Novex Bis-Tris gels (Life Technologies, Grand Island, NY) and immobilized on a nitrocellulose membrane. The membranes were blocked with Odyssey Blocking Buffer (LI-COR Biotechnology, Lincoln, NE) for 1 hour at room temperature, then probed with primary antibody diluted in Odyssey Blocking Buffer overnight at 4°C. Membranes were washed 5 times for 5 min each in Tris-buffered saline + 0.05% Tween-20 (TBST) solution, and then probed with secondary antibody in Odyssey Blocking Buffer for 1 hour at room temperature. After secondary labeling, membranes were washed 5 times for 5 min in TBST. Finally, to determine even protein loading, the membranes were probed with GAPDH-800 labeled antibody for 1 hour at room temperature. Washes were repeated as above and membranes imaged using a LI-COR Odyssey scanner.

IDUA Enzymatic Activity Assay: IDUA enzymatic activity in plasma and tissue protein extracts was assessed in a fluorometric assay using the synthetic substrate 4-methylumbelliferyl alpha-L-iduronide (4-MU-iduronide; Glycosynth, Warrington, Cheshire, England). Tissue protein extracts were prepared by mixing mouse tissue samples with 1 mL of PBS (pH 7.4). Samples were stored on ice, and homogenized with a Polytron® Homogenizer (Kinematica, Lucerne, Switzerland) at a speed level of 5 for 5 seconds. Homogenization was repeated 3 times, or until no solid tissue particles were visible. Following homogenization, 11 µL of 10% Triton X-100 was added to each sample. Homogenates were then incubated on ice for 10 minutes, after which crude homogenates were centrifuged at 3,000 rpm for 10 minutes to clear the lysate prior to analysis. Protein concentrations in tissue extracts were determined using PierceTM 660 nm Protein Assay Reagent (Thermo Fisher Scientific), according to the manufacturer's instructions.

In the IDUA enzymatic activity assay, 25 µL of mouse tissue protein extract or mouse plasma sample were mixed with 25 µL of 360 µM synthetic substrate (4-MU-iduronide) dissolved in formate buffer (0.4 M sodium formate, pH 3.5, 0.2% Triton X-100). Reactions were incubated at 37°C for 30 minutes and terminated by addition of 200 µL of glycine carbonate buffer (84 mM glycine, 85 mM anhydrous sodium carbonate). The release of 4-MU was determined by measurement of fluorescence (Ex365/Em450) using a BioTek microplate reader (BioTek, Winooski, VT). A standard curve was generated using dilutions of 4-MU (Sigma-Aldrich, St. Louis, MO). The resulting data were fitted with a linear curve and the concentration of 4-MU in test samples was calculated using this best-fit curve. Enzymatic activity is expressed as nmol 4-MU released per hour of assay incubation time, per mL of plasma or mg of tissue extract (nmol/hr/mL or nmol/hr/mg).

Assessment of GAG Levels in Urine and Mouse Tissues: GAG levels in tissues and urine were determined using the BlyscanTM Glycosaminoglycan Assay Kit (Biocolor, Carrickfergus, County Antrim, United Kingdom), according to the manufacturer's instructions. Prior to GAG assay, mouse tissue homogenates, prepared as described above, were mixed with proteinase K (20 mg/mL) at a ratio of 1:3 and digested at 55°C for 24 hours, followed by heat inactivation of the proteinase K by boiling for 10 minutes. Tissue homogenates were next digested with 200 units of DNase and 2 µg of RNase at room temperature for 24 hours while shaking. DNase and RNase were then heat inactivated by boiling for 10 minutes, and the resultant tissue homogenates were used for GAG assay.

Histopathological analysis: Terminal body and organ (adrenal glands, brain, heart, kidney, liver, spleen, testes or ovaries) weights were collected at the Day 120 necropsy for males (Group 1 [n=5], Group 2 [n=4], Group 4 [n=4], and Group 6 [n=2]) and females (Group 3 [n=4], Group 5 [n=5], and Group 6 [n=2]), and group means and standard deviations were routinely calculated in Excel.

Animals from Groups 1 through 6 were necropsied and tissues collected were placed into 10% neutral buffered formalin (NBF) for fixation. The following tissues were trimmed to produce histological sections: adrenal glands, aorta, brain, cecum, cervix, colon, duodenum, epididymis, esophagus, eyes, femoral-tibial joint, gallbladder, Harderian gland, heart, ileum, injection site, jejunum, kidney, larynx, liver, lungs with bronchi, lymph node (mesenteric), optic nerve, ovaries, pancreas, parathyroid gland, pituitary gland, prostate, rectum, salivary gland, sciatic nerve, seminal vesicles, skeletal muscle (quadriceps femoris), skin, spinal cord (cervical, lumbar, thoracic), spleen, sternum with bone marrow, stomach, testes, thymus gland, thyroid gland, tongue, trachea, urinary bladder, uterus, and vagina. Tissues listed above, from animals in all dose groups, were routinely processed, and embedded in paraffin blocks. The blocks were sectioned and stained with hematoxylin and eosin (H&E). Slides were evaluated microscopically by a board-certified veterinary pathologist.

### B. Results

Gene Modification Analysis: Percent Indels at Albumin Locus: To evaluate the activity of ZFN constructs in targeted hepatocytes in vivo, genomic DNA was isolated from livers of all mice at necropsy. The ZFN target site at the mouse albumin intron 1 locus was subjected to sequence analysis using the MiSeq system (Illumina, San Diego CA). ZFN activity was then determined by the presence of small insertions and/or deletions (indels), which are the hallmarks of ZFN activity. ZFN activity was detected in all groups of mice receiving surrogate mouse ZFNs + SB-mu-IDUA (Groups 4 and 5). The levels of albumin gene modification (% indels) in liver tissue at 1 month were 46.67 ± 5.74% and 34.05 ± 2.06% (mean ± SD) for male and female animals, respectively. The levels of gene modification at 4 months were 55.65 ± 4.08% and 45.69 ± 2.26% for male and female animals, respectively. There were no levels of ZFN cutting above background in the formulation buffer control groups or the Donor Only groups at either time point.

To confirm the specificity of the liver-specific promoter, MiSeq analyses were also performed on genomic DNA from the spleen, heart, and muscle. These organs were chosen as they showed the highest levels of IDUA activity outside of the liver in ZFN+Donor-treated mice (see Figure 1A). These analyses were performed on mice from Group 4 and Group 5, because they showed the highest level of ZFN activity in the on-target tissue. The corresponding untreated male mice (Group 2) were included as a negative control. No gene modification was observed in the spleen and heart (non-target tissues) of Groups 2 and 4 at 4 months, confirming the specificity of the liver-specific promoter and demonstrating that IDUA activity and GAG reduction found in these tissues is not derived from ZFN activity and donor integration outside of the liver.

hIDUA Western Blot. After confirmation of appropriate gene modification of the mouse albumin locus in hepatocytes, protein extracts from liver were next examined for hIDUA expression from the albumin locus. To avoid potential background signal from endogenous mouse IDUA, Western blot analyses were performed using a detection antibody raised against human IDUA.

As shown in Figure 1B, hIDUA was detectable at 1 and 4 months in the livers of all male and female mice receiving mouse surrogate ZFNs + hIDUA donor. Expression appeared to be increased at 4 months. Wild type control mice injected with formulation buffer alone showed no hIDUA signal, demonstrating the species-specificity of the antibody used. The expression of hIDUA was dependent upon the presence of both ZFNs and the hIDUA donor, as no hIDUA signal was observed in the absence of ZFNs (Donor Only group).

IDUA Enzymatic Activity. To determine whether the hIDUA transgene that was expressed from the liver albumin locus produced functionally active protein, a fluorescence-based enzymatic activity assay was performed for IDUA. This assay was performed on 1) protein extracts from the liver (to ensure that expressed enzyme was active); 2) plasma (to determine whether expressed enzyme was being secreted from hepatocytes); and 3) protein extracts from the brain, heart, lung, muscle, and spleen (to determine if enzyme was being expressed in a form that is competent to be taken up by secondary tissues).

At 1 month, IDUA activity in the liver of ZFN+Donor-treated mice (65.26 ± 24.68 and 40.54 ± 14.54 nmol/hr/mg for male and female animals, respectively) was 9 to 10 times higher than the levels of activity found in wild type mice (4.09 ± 0.99 nmol/hr/mg). IDUA activity in ZFN+Donor-treated mice was also markedly increased compared to activity in both control (0.11 ± 0.02 [male] and 0.16 ± 0.04 [female] nmol/hr/mg) and Donor Only (0.18 ± 0.05 nmol/hr/mg) MPS I mice. The low levels of activity found in MPS I mice receiving the SB-mu-IDUA donor in the absence of ZFNs (Donor Only) demonstrate that both ZFNs and SB-mu-IDUA donor are required for integration and expression of hIDUA in mouse hepatocytes.

Liver IDUA activity was further increased in the ZFN+Donor-treated mice at 4 months (147.32 ± 65.86 and 63.59 ± 43.42 nmol/hr/mg for male and female animals, respectively), compared to activity in wild type animals (8.05 ± 1.55 nmol/hr/mg).

As shown in Figure 2A, plasma IDUA enzymatic activity in ZFN+Donor treated mice was also increased compared to the levels observed in control and Donor Only mice, starting at Day 14 and throughout the study. Average IDUA activity remained elevated ∼1.5-10 fold above endogenous, physiological levels observed in wild type control mice from Day 14 until the end of the study. IDUA activity was compared at 1 month and 4 months in the brain, heart, liver, lung, muscle and spleen (Figure 2C) and the IDUA activity at 4 months in the ZFN+Donor groups showed significantly higher activity than gender-matched, untreated MPS I mice in the heart, liver, lung, and spleen (males and females) and muscle (males only).

In MPS I mice receiving both surrogate mouse ZFNs + hIDUA donor, IDUA activity in all non-target tissues, except the brain at 1 month, approached levels of activity in the wild type mice, demonstrating that the IDUA produced at the albumin locus in hepatocytes was secreted into the plasma, from which it was taken up by secondary tissues in an active form (see, Figure 4). In the brain an increase in IDUA activity was detected, corresponding to approximately 5% of IDUA activity found in the brains of wild type mice (see, Figure 4E).

Assessment of GAG Levels in Urine and Mouse Tissues: To determine whether the observed correction in the IDUA enzyme deficiency in the ZFN+Donor-treated MPS I mice was associated with prevention of increased levels or reductions over time in the disease biomarker glycosaminoglycan (GAG), GAG levels were determined in urine and tissue extracts of all animals.

The results indicated that urinary GAG levels were increased throughout the study as disease progressed, in the MPS I mice receiving formulation buffer, as compared to levels in wild type mice. As shown in Figure 3C, treatment with both surrogate mouse ZFNs and hIDUA donor in male and female MPS I mice prevented the increase in urinary GAG levels seen in the untreated MPS I mice by Day 14 post-dosing. GAG levels were also markedly increased in all tissues (except brain) of formulation buffer-treated male and female MPS I mice compared to wild-type mice (see, Figure 5). These levels were completely normalized in ZFN+Donor-treated MPS I animals. Normalization occurred both in the liver (where IDUA was produced from the albumin locus) and in secondary tissues (which must have taken up IDUA from the plasma). In the brain the GAG levels were lower in the treated animals as compared to the untreated animals (see, Figure 5E).

This data showed that liver-produced hIDUA entered the circulation (plasma) and was taken up by the spleen, heart, liver, lung, and skeletal muscle, and led to reduction in GAG levels in secondary tissues in ZFN+Donor-treated MPS I mice.

### Histopathological Assessment

### Interim and terminal necropsies, Study Day 28 and 120

A subset of tissues (brain, lung, heart, liver, skeletal muscle and spleen) were evaluated from the Day 28 necropsy.

The wild-type C57BL/6 control animals showed no test article-related pathologic findings. Male and female MPS I control mice developed vacuolation of various cell types throughout the body, and this was interpreted as lysosomal accumulation of glycoaminoglycans (GAGs). Vacuolation is a characteristic microscopic appearance of lysosomes engorged with glycoaminoglycans (Ohmi et al (2003) Proc Natl Acad Sci USA 100(4):1902-1906). When compared with male and female MPS I control mice at Day 28, animals at Day 120 had an increased incidence and/or severity of typical vacuolation of various cell types throughout the body that was considered to be lysosomal accumulation of GAGs. The wild-type C57BL/6 control animals showed no test article-related pathologic effects on Days 28 or 120.

CNS Tissue Evaluation. When compared with MPS I controls, animals treated with ZFNs+hIDUA donor or hIDUA donor-only had similar incidence and/or severity of large neuron or glial vacuolation in the brain. However, in the spinal cord, males given ZFNs+hIDUA donor had decreased incidence and/or severity of neuronal or glial vacuolation. Likewise, females given ZFNs+hIDUA donor had decreased incidence and/or severity of neuronal or glial vacuolation, except for glial vacuolation in the lumbar spinal cord of females.

Non-CNS Tissue Evaluation. The severity of aortic vacuolation (tunica media) was decreased in animals given ZFNs+hIDUA donor when compared with MPS I or hIDUA-only controls (minimal-to-mild and moderate, respectively). When compared with MPS I controls, there was a decreased incidence and/or severity of vacuolation in the lungs, heart valve or interstitium, aortic tunica media, renal tubular epithelium, urinary bladder (urothelium and interstitium), skeletal muscle interstitial cells, splenic macrophages/ histiocytes, mesenteric lymph node macrophages/histiocytes, thymic interstitial cells, Kupffer cells, sciatic nerve, parathyroid cells, corneal stromal/endothelial cells (Descemet's membrane), gastrointestinal tract (tunica muscularis and interstitium [virtually absent in females]), pituitary pars distalis, and injection site of animals given ZFNs+hIDUA donor (Groups 4 and 5). In females given ZFNs+hIDUA donor (Group 5), there was a decreased incidence of vacuolation of sternal or femorotibial joint chondrocytes/osteocytes/periosteal cells, femorotibial joint synovium, and vacuolation in the ovaries, uterus, cervix, and vagina. In males given ZFNs + hIDUA donor, a decreased incidence of epididymal epithelial or interstitium cell vacuolation was present.

Neurobehavioral Assessment: Barnes Maze. Cognitive performance of the mice was tested during the final week prior to necropsy using the Barnes Maze Test. MPS I mice have previously shown leaning deficits in both the Barnes maze (Belur et al (2015). Presented at American Society of Gene and Cell Therapy ASGCT May 13-16; New Orleans, LA) and other tests of cognitive ability, such as the water T-maze (Ou *et al. ibid*).

Barnes Maze testing occurs on a circular platform with forty holes ringed around the center of the platform. *See,* Figure 6. All the holes are blocked except for one hole which has an "escape box" that the mouse can access from the hole. Bright overhead lighting creates an aversive stimulus, encouraging the animal to seek out the target escape hole and avoid the light. Visual cues within easy sight are placed on the walls around the maze and act as spatial cues to orient the mice (see Figure 6). The mice were trained on the Barnes maze for 6 days, at 4 trials a day, with a maximum time limit of 3 minutes per trial. Intervals were 12-15 minutes between consecutive trials for each animal.

As shown in Figure 7A and 7B for the male and female animals, respectively, wild type male mice improved over time, as indicated by decrease in latency to find the target escape hole over the 6 days of the test, while the escape latencies did not improve in untreated MPS I male mice over time (p<0.05 vs. wild type on Days 4-6). In contrast, male MPS I mice receiving surrogate mouse ZFNs + hIDUA donor showed an improvement in maze performance over time as compared to the formulation buffer-treated MPS I group (p<0.05 on Days 4-6), and perform equally well as the wild type animals. By Day 4 of the test, ZFN+Donor-treated male MPS I mice showed a statistically significant difference in learning behavior with an average on Day 6 of 53 ± 26 (mean ± SEM) seconds to find the target hole, whereas untreated male MPS I mice took 149 ± 17 seconds to find the target. Wild type male mice reached the target in 51 ± 15 seconds on Day 6.

The female MPS I mice receiving formulation buffer did not show a significant difference in performance over the 6 testing days, compared to wild type mice. However, the female MPS I mice treated with ZFNs + Donor showed a trend towards better maze performance during the course of the study. On Day 6, treated female MPS I mice took 38 ± 23 seconds to find the target hole, and formulation buffer-treated female MPS I mice took 96 ± 33 seconds to find the target. These results suggest that hIDUA may be gaining access to the CNS in MPS I mice and producing positive neurobehavioral effects without modifications to the hIDUA protein to facilitate crossing of the blood-brain barrier. (*see, e.g.,* Ou *et al. ibid*). Therefore, the consistently high plasma levels of IDUA achieved herein (due to constant secretion from the liver) resulted in the unmodified IDUA crossing the blood-brain barrier.

Importantly, the GAG data indicated that expressed IDUA activity in ZFN+Donor-treated MPS I mice was associated with a normalization of urinary GAG levels, as well as reduction of GAG levels in liver and secondary tissues. Furthermore, the treatment was associated with trends in a decrease in vacuolation in CNS and other target tissues as well as an improvement in cognitive performance (improved spatial memory) in the Barnes Maze test.

In summary, the data presented here demonstrate that ZFN-mediated gene replacement at the albumin locus for MPS I subject treats the disease. This 4-month pharmacology/toxicology study demonstrated that: co-delivery of surrogate mouse ZFNs with a hIDUA transgene donor via liver-tropic rAAV2/8 vectors leads to 1) efficient modification of the albumin locus in MPS I mouse hepatocytes in vivo; 2) high levels of hIDUA enzyme expression and activity in the liver; 3) secretion of functional enzyme into the plasma; and 4) uptake of IDUA from the plasma by secondary tissues, including spleen, lung, heart, muscle and brain. Moreover, the results also demonstrate that the levels of IDUA activity achieved are sufficient for at least a partial correction of the metabolic defect (GAG accumulation) in non-target tissues, which results improved behavioral function.

### Example 3: Treatment of MPS II (IDS knockout or Ids Y/-) in a mouse model

Similar to the work done above for treatment of MPS I using an IDUA transgene, the experiments were repeated in a MPS II mouse model system using a corrective human IDS transgene. AAV2/8 virus were made comprising the IDS transgene and used to treat the MPS II mice along with the mouse ZFN rAAV vectors (SB-48641, SB-31523, shown in U.S. Patent Application 14/872,537) targeting a homologous site in intron 1 of the mouse albumin gene as well as a donor that encodes a promoterless human IDS transgene (hIDS) flanked by arms with homology to the mouse locus (SB-mu-IDS). The ratio of ZFN:ZFN:Donor used in this study was 1:1:8. For this mouse study, the surrogate reagents were packaged and delivered using serotype rAAV2/8, as it confers superior transduction and faster transgene expression than rAAV2/6 vectors in mice *in vivo.* The rAAV2/8 vectors were diluted into the formulation buffer, phosphate-buffered saline (PBS) supplemented with 35 mM NaCl and 5% glycerol, pH 7.1.

The study design is shown below in Table 5 which lists the 6 groups of mice and the amount of AAV2/8 virus of each type used.

**Table 5: MPS II Study Design**

| **Group** | **Gender** | **Genotype** | **Treatment** | **ZFN (each)** | **Donor** |
|---|---|---|---|---|---|
| **1** | Male | Wildtype | *Formulation control* | N/A | N/A |
| **2** | Male | MPS II | *Formulation control* | N/A | N/A |
| **3** | Male | MPS II | *ZFN+Donor low dose* | 2.50E+10 | 2.00E+11 |
| **4** | Male | MPS II | *ZFN+Donor mid dose* | 5.00E+10 | 4.00E+11 |
| **5** | Male | MPS II | *ZFN+Donor high dose* | 1.50E+11 | 1.20E+12 |
| **6** | Male | MPS II | *Donor only mid dose* | N/A | 4.00E+11 |

This study was carried out essentially as described above for the MPS I treatment. Western blots were run as described above and the expressed human IDS protein was detected using an antibody raised against human IDS protein to avoid detection of the mouse protein (AF2449 from R&D Systems). The results showed that human IDS protein was detectable in the treated groups that received both the ZFNs and the donor (groups 3, 4, and 5 above) at 1 month and at 4 months.

IDS activity was detected by lysing the tissue to be analyzed followed by detection of the IDS activity. The methodology followed is below:

Tissue lysis: Tissue protein extracts were prepared by mixing mouse tissue samples with saline (0.9% sodium chloride). Samples were stored on ice, and homogenized using a Bullet Blender® Homogenizer (Next Advance, Averill Park, NY). Homogenization was performed until no solid tissue particles were visible. Following homogenization, crude homogenates were centrifuged at maximum speed for 15 minutes at 4°C to clear the lysate prior to analysis. Protein concentrations in tissue extracts were determined using PierceTM 660 nm Protein Assay Reagent (Thermo Fisher Scientific), according to the manufacturer's instructions.

IDS Activity Assay: IDS enzyme activity was assessed in a fluorometric assay using the synthetic substrate 4 methylumbelliferyl α L idopyranosiduronic acid 2 sulfate (4 MU IDS; Santa Cruz Biotechnology).

This is a two-step assay, described by Voznyi et al. ((2001), J. Inherit Metab Dis 24(6): 675-80). In the first step, 4 MU IDS is incubated with test samples; if IDS is present, it catalyzes the removal of the sulfate group from the substrate. The resulting compound, 4 methyl umbelliferyl α L iduronide (4 MU iduronide), is a substrate for a second enzyme, α L iduronidase (IDUA). After the first step has been incubated for a fixed time, IDS activity is quenched and excess recombinant IDUA is added, to drive the cleavage of 4 MU iduronide, yielding the fluorescent compound 4 methylumbelliferone (4 MU). Briefly, 10 µL of diluted sample was mixed with 20 µL of 1.25 mM 4 MU IDS dissolved in substrate buffer (0.1 M Na acetate, pH 5.0, 10 mM Pb(II) acetate). Reactions were then incubated at 37°C.

To halt IDS activity and to convert any de sulfated substrate to 4 MU, 20 µL of 2x concentrated McIlvaine's buffer (0.2 M citrate, 0.4 M phosphate, pH 4.5) was added to each sample, followed by 10 µL of 1 µg/mL of recombinant IDUA. IDUA reactions were incubated overnight at 37°C, and then terminated by addition of 200 µL stop solution (0.5 M NaHCO3 / Na2CO3, pH 10.7, 0.2% Triton X 100). The release of 4 MU was determined by measurement of fluorescence (Ex365/Em450) on a SynergyTM MX microplate reader (BioTek, Winooski, VT).

A standard curve was generated using 4 MU (Sigma Aldrich, St. Louis MO). The resulting data were plotted, a log log curve was fitted to the data, and the concentration of 4 MU in test samples was calculated using this best fit curve. Enzymatic activity was expressed as nmol 4 MU released per hour of assay incubation time (first reaction step only), per mL of plasma or mg of protein lysate (nmol/hr/mL or nmol/hr/mg).

IDS protein was measured in the livers of the animals at 1 month and 4 months post-treatment (Figure 8A). The data demonstrated that the protein was detectable in all groups that received both the donor and the ZFNs. In addition, activity was measured in the plasma of the treatment groups over time, and activity was detected in all samples from groups receiving ZFN and donor. Highest activity was detected at the high dose groups (Figure 8B). At the 4 month sacrifice time point, IDS activity was also detected in all tissues from the animals receiving the ZFN and donor. Activity was also measured in a number of organs of the animals in the study (Figure 8C), and the high dose treatment group shows significantly more IDS activity as compared to the untreated MPS II mice in all tissues tested, including brain.

Tissue GAG levels were measured as described in Example 2 and the results showed a significant reduction of GAG in all tissues analyzed from the high dose animals except for the brain (Figures 9A and 9B).

Cognitive performance of the treatment groups was tested using the Barnes maze test 4 months post-treatment (Figure 10). Data represent mean ± SEM of the time it took the animals to find the target (average of 4 trials each day) over 6 days of testing. The data for each individual mouse is presented below in Table 6 (expressed as time in seconds to reach to hole for each mouse on each test day). The data for individual mouse is shown across the table in a horizontal direction (where each mouse is numbered 1 through 10, and each data point is the average of the four runs done per trial day) and each trail (trial days 1-6) is shown vertically. At the high treatment dose, there was a significant difference between those animals receiving the treatment and the untreated MPS II animals.

**Table 6: Cognitive performance of MPS II treatment groups in the Barnes maze (seconds)**

| Trial ↓ | Mouse number → | | | | | | | | | |
|---|---|---|---|---|---|---|---|---|---|---|
| | **Wild type, Untreated (n=10)** | | | | | | | | | |
| | **1** | **2** | **3** | **4** | **5** | **6** | **7** | **8** | **9** | **10** |
| **1** | 180 | 180 | 156.75 | 180 | 180 | 180 | 138 | 145.25 | 97.25 | 180 |
| **2** | 157.25 | 180 | 135.25 | 120.25 | 136.5 | 121.5 | 103.75 | 83.75 | 47 | 151.5 |
| **3** | 36 | 150 | 123.75 | 64 | 140 | 68.75 | 29.75 | 31.75 | 15.75 | 107.25 |
| **4** | 102 | 88 | 98.75 | 38.75 | 87.5 | 20 | 10.25 | 22.75 | 13 | 56 |
| **5** | 11.5 | 96.5 | 23.5 | 28 | 28 | 8.25 | 14.5 | 12.75 | 17 | 37.75 |
| **6** | 14.25 | 30 | 55 | 9 | 14.25 | 10.75 | 32.25 | 17.25 | 13.25 | 33.25 |
| | | | | | | | | | | |

| | **MPS II, Untreated (n=10)** | | | | | | | | | |
|---|---|---|---|---|---|---|---|---|---|---|
| | **1** | **2** | **3** | **4** | **5** | **6** | **7** | **8** | **9** | **10** |
| **1** | 168 | 79.75 | 126 | 180 | 180 | 180 | 180 | 148.5 | 174.25 | 180 |
| **2** | 167.5 | 49.25 | 76.75 | 81.25 | 79.75 | 122.5 | 55.25 | 46.25 | 37.25 | 103.25 |
| **3** | 101 | 90.5 | 34 | 12.75 | 35.25 | 59.25 | 33 | 40.75 | 81.25 | 121.75 |
| **4** | 137.75 | 30.5 | 83.75 | 97.25 | 89.75 | 65.5 | 50 | 38.25 | 63.75 | 107 |
| **5** | 99.25 | 23.25 | 30.5 | 66 | 114.25 | 56 | 29.25 | 14.5 | 34.75 | 79 |
| **6** | 106.75 | 15 | 63 | 16.75 | 74.25 | 127.75 | 42 | 55.25 | 62.5 | 106.75 |
| | | | | | | | | | | |

| | **MPS II, ZFN+Donor High (n=10)** | | | | | | | | | |
|---|---|---|---|---|---|---|---|---|---|---|
| | **1** | **2** | **3** | **4** | **5** | **6** | **7** | **8** | **9** | **10** |
| **1** | 180 | 180 | 82.75 | 180 | 85.75 | 158.25 | 94.25 | 144.75 | 180 | 116.5 |
| **2** | 54.5 | 180 | 51.75 | 73.5 | 31.5 | 68.5 | 50.5 | 59.25 | 67.25 | 44.5 |
| **3** | 109.25 | 152.75 | 49 | 53.25 | 23.5 | 25.5 | 25.5 | 34.5 | 47.75 | 12.5 |
| **4** | 31 | 117.25 | 22.25 | 69.5 | 26.75 | 14.75 | 11.75 | 27.25 | 30.75 | 16.75 |
| **5** | 34 | 30 | 24.75 | 94.5 | 17 | 17.5 | 14 | 15 | 29 | 21.5 |
| **6** | 17 | 29.5 | 21 | 113.5 | 25.25 | 28.25 | 17.75 | 10.75 | 13.5 | 30.75 |

As shown, at the high treatment dose, there was a significant difference between those animals receiving the treatment and the MPS II animals.

### Example 4: Characterization of the IDS and IDUA protein produced in human cells

### A. Generation and Characterization of HepG2 Cells (Pool) and HepG2 Subclones

HepG2 cells transduced with the hALB ZFNs SBS# 47171 and SBS# 47931 essentially as described in WO 2015/089077 and integration of the IDS or IDUA donor were assessed by MiSeq analysis 3 days post transduction and showed 59.27% indels (hIDS) and 62.27% indels (hIDUA) at the albumin locus. Based on this high level of modification, these cells were selected for subcloning and four hIDS subclones (numbers 4, 8, 15 and 55) and three hIDUA subclones (numbers 21, 25 and 30) were then were seeded in 24 well plates and supernatants were analyzed for hIDS or hIDUA secretion by both IDS or IDUA enzyme activity assay and ELISA (Figure 11). HepG2 cells transduced with the SB 47171 and SB 47931 ZFN vectors alone were used as a negative control ("ZFN only" in Figure 11).

The four IDS subclones displayed secretion of functional IDS, with activity levels in the supernatant of these subclones ranging from 408 to 1,855 nmol/hr/mL. All subclones showed several fold higher hIDS activity than the original HepG2 pool ('IDS-pool' in Figures 11A and 11B).

The three IDUA subclones displayed secretion of functional IDUA, with activity levels in the supernatant of these subclones ranging from 92-475 nmol/hr/mL (Figures 11C and 11D).

These results were substantiated by an hIDS or hIDUA ELISA. For hIDS, all four subclones also showed hIDS secretion into the cell culture supernatant at levels ranging between 156 to 438 ng/mL, several fold higher than the original HepG2 pool. In both assays, no background was seen in the HepG2 pool transduced with ZFN only, indicating that HepG2 cells do not secrete endogenous hIDS at a level detectable by these assays. Similarly, for hIDUA, all three subclones also showed hIDUA secretion into the cell culture supernatant at levels ranging between 40.6-209.8 ng/mL. In both assays, no background was seen in the untreated HepG2 cells or the HepG2 subclones transduced with ZFN only, indicating that HepG2 cells do not secrete endogenous hIDUA at a level detectable by these assays.

### B. Characterization of SB IDS Integration at the Albumin Locus in HepG2 Subclones by Taqman®

HepG2 subclones 4, 8 15 and 55 were further characterized with respect to the modification at the genomic albumin locus. First, to determine the mode of integration of the IDS ("SB-IDS") donor either by NHEJ or HDR, a Taqman® assay was developed with primers and probes specific for each method of integration at the 5' end of the transgene (Figure 12). After integration by NHEJ, the AAV ITRs (and homology arms) are still present and are recognized by a NHEJ specific probe, which leads to a positive signal in the Taqman® assay (Figure 12A). In contrast, if the SB-IDS donor is integrated by HDR, the ITRs are missing which leads to a negative signal with these reagents. Reagents detecting SB-IDS donor integrated by HDR consist of PCR primers optimized for a 429 bp product and a probe that binds to Exon 1 of albumin (Figure 12 B). If these HDR reagents are used on a SB-IDS donor integrated by NHEJ, the resulting 979 bp PCR product would be amplified inefficiently due to the additional size, leading to a negative signal.

The results showed that all four analyzed ZFN+Donor treated subclones showed signal by NHEJ or HDR specific reagents, confirming the 5' integration events of the SB-IDS transgene at the albumin locus in these subclones. Results for subclones 15 and 55 clearly indicated HDR as the mechanism for SB-IDS integration (Figure 12C). Subclones 4 and 8 consistently scored positive for an NHEJ integration event in all three Taqman® assays. However, subclones 4 and 8 also scored positive for integration by HDR in 1 out of the 3 Taqman® assay repeats. In the other two repeats, these subclones were either negative for HDR integration or there was a large amount of variation among replicates in the assay, making the method of integration difficult to determine. Thus, these samples were considered to be negative for HDR. Based on indel analysis of the second albumin allele, we concluded that subclones 4 and 8 are 88% and 83% clonal, respectively, suggesting that the low and inconsistent HDR signal may result from the presence of a minor HepG2 population with an HDR based integration of SB-IDS in these subclones.

### C. Characterization of SB-IDUA Integration at the Albumin Locus in HepG2 Subclones by Taqman®

HepG2 IDUA subclones 21, 25 and 30 were further characterized with respect to the modification at the genomic albumin locus. First, to determine the mode of integration of the IDUA ("SB-IDUA") donor either by NHEJ or HDR, a Taqman® assay was developed with primers and probes specific for each method of integration at the 5' end of the transgene (Figure 12). After integration by NHEJ, the AAV ITRs (and homology arms) are still present and are recognized by a NHEJ specific probe, which leads to a positive signal in the Taqman assay (Figure 12E). In contrast, if the SB-IDUA donor is integrated by HDR, the ITRs are missing which leads to a negative signal with these reagents. Reagents detecting SB-IDUA donor integrated by HDR consist of PCR primers optimized for a 429 bp product and a probe that binds to Exon 1 of albumin (Figure 12F). If these HDR reagents are used on a SB-IDUA donor integrated by NHEJ, the resulting 979 bp PCR product would be amplified inefficiently due to the additional size, leading to a negative signal.

The results showed that all three analyzed ZFN+Donor treated subclones showed signal by NHEJ or HDR specific reagents, confirming the 5' integration events of the SB-IDUA transgene at the albumin locus in these subclones. While the results for subclone 21 were clearly indicating hIDUA integration by HDR, the mechanism of integration for subclones 25 and 30 was unclear. Therefore, an alternative genotyping method was carried out for all three subclones. For this genotyping, genomic DNA from subclones 21, 25 and 30 was subjected to a radiolabeled PCR assay to assess the presence of 5' transgene integration events at the ZFN target site within the albumin locus (Figure 12H). The resulting gel confirmed HDR integration for clone 21, NHEJ for clone 25 and NHEJ for clone 30 (Figure 12I). Arrows point to band regions characteristic of insertion via NHEJ (upper arrow) or HDR (lower arrow).

### D. Characterization of hIDS Polypeptides Produced from Albumin Locus in HepG2 IDS Subclones

The expression *in vivo* of the IDS in the liver and then subsequence uptake by the target tissue is dependent upon the glycosylation pattern on the IDS protein (Figure 13). To further characterize the hIDS protein produced from the albumin locus, Western blotting was performed on supernatants generated from HepG2 IDS and control HepG2 clones. Two sets of hIDS-expressing HepG2 cells were used for this analysis. The first is a high IDS expressing mixed clone, generated in the same manner as the HepG2 IDS clones analyzed above. MiSeq analysis at the albumin locus showed that this 'mixed clone' consisted of 69.9% unmodified HepG2 cells, 21.4% of a HepG2 IDS subclone containing a 5bp deletion in the non-IDS insert albumin allele, and of three other subclones containing small deletions which together represented 6.8% of the total population (labelled "Mix" in Figure 14).

The second HepG2 IDS clone used was clone #55, a lower expressing IDS clone characterized above. A HepG2 IDUA clone was used as a negative control, as it had been generated in the same manner as these HepG2 IDS clones, but with IDUA (the gene deficient in MPS I) integrated at albumin rather than IDS ('Ctrl' in Figure 14).

Human IDS is endogenously expressed initially as a 550 amino acid immature precursor form, containing both a signal peptide and a propeptide. Both the 25 amino acid signal peptide and the 8 amino acid propeptide are cleaved during post translational modification and maturation of the hIDS protein (Wilson et al., (1990) Proc Natl Acad Sci USA. 87(21) 8531 5). The resultant 73- 78 kDa polypeptide is phosphorylated and glycosylated in the Golgi apparatus into a 90 kDa precursor, which is subsequently cleaved in the lysosome into a 55 kDa intermediate, and finally a 45 kDa mature hIDS protein, with the release of an 18 kDa polypeptide (Froissart et al., (1995) Biochem. J. 309(Pt 2): 425-30).

To determine which of these forms of hIDS were produced from the albumin locus following hIDS integration, Western blots were performed on HepG2 cell pellets, HepG2 cell culture supernatants (following hIDS secretion from the cell), and on unmodified primary hepatocyte pellets following incubation with this HepG2 cell culture supernatant (mimicking uptake by a secondary tissue).

As shown in Figure 14, both full-length IDS and all of the intermediate and mature polypeptide forms were found in the HepG2 pellets that produce hIDS from Albumin ('HepG2 Pellet'). The full-length form of IDS was seen as a smeary band, likely due to on-going post translational modification (glycosylation) of newly synthesized IDS in these producer cells. This demonstrated that hIDS protein produced from the albumin locus in hepatoma HepG2 cells was post- translationally modified and secreted similar to the naturally occurring polypeptide. The migration of the produced enzyme around 90 kDa was consistent with that of recombinant IDS produced in fibroblasts (Froissart *et al., ibid*) and CHO cells (Elaprase® and GREEN CROSS recombinant IDS; see US Patent Publication No. 20130236442).

Second, analysis of conditioned supernatant from these HepG2 subclones indicated that full-length hIDS was the primary form of IDS secreted from the cell, with only a very weak band for the 18 kDa band also detected ('HepG2 Supernatant'). When this conditioned cell culture supernatant was incubated with naive, unmodified primary hepatocytes, these hepatocytes efficiently take up hIDS from the supernatant ('Primary Hepatocytes Pellet'). Although full-length hIDS was detectable in these pellets, it was clear that hIDS was readily able to be processed to the mature forms of the protein as well, as the 55, 45, and 18 kDa polypeptides were all present in these pellets. Finally, the hIDS taken up from the supernatant is active, as there was a 6.5 10.3 fold increase in hIDS activity in primary hepatocytes incubated with supernatant from hIDS secreting HepG2 cells.

Combined, these data demonstrate that hIDS produced from albumin was normally processed to its mature forms in the cell of origin, the full-length form was secreted from the cell, and this full-length hIDS was taken up by secondary cells and processed efficiently into its mature and active forms.

### E. Characterization of Glycosylation of hIDS and hIDUA Produced from Albumin Locus in HepG2 Subclones

It has been suggested/demonstrated that Mannose 6 Phosphate modification on the IDS or IDUA enzymes is necessary for efficient cellular uptake and proper lysosome targeting in several cell types like hepatocytes, neurons and glia cells (Daniele et al., (2002) Biochim Biophys Acta. 1588(3):203-9). Therefore, in order to characterize the glycosylation pattern of hIDS and hIDUA produced and secreted from the albumin locus, Western blotting was performed on HepG2 IDS or IDUA subclone supernatants. IDS contains 8 potential N glycosylation sites, which are occupied by a combination of high mannose, hybrid, and complex oligosaccharide chains (Froissart et al., *ibid*). These types of glycosylations can be distinguished using specific glycosidases: peptide-N-glycosidase F (PNGase F) is able to cleave all 3 types of oligosaccharides from glycoproteins, whereas endoglycosidase H (Endo H) is only able to cleave high mannose and some hybrid oligosaccharides, but not complex oligosaccharides, from N-linked glycoproteins (Figure 15A).

To determine which species of glycosylations were present on hIDS produced and secreted from the albumin locus, cell culture supernatants were harvested from two different HepG2 IDS populations (Mix and Clone 55; described above) and on control supernatant harvested from a HepG2 subclone with IDUA integrated at albumin (Control). Supernatants were left undigested (U) or subjected to digestion with either PNGase F (P) or Endo H (E). Figure 15B shows that PNGase F digestion (P) of supernatant from either HepG2 IDS population resulted in a complete shift to a single band at approximately 60 kDa, indicating removal of all oligosaccharide chains from the protein (= non-glycosylated hIDS). By contrast, Endo H digestion (E) resulted in a smeary band of intermediate mobility. Control supernatants did not show any hIDS expression, indicating that little to no hIDS was secreted from untreated cells.

Similarly, when the same set of glycosidase digestions were performed on protein lysates from HepG2 pellets (Figure 15C), the three major large IDS forms seen (full-length ∼73- 78 kDa; intermediate ∼55 kDa; and mature ∼45 kDa) all showed the same shifts in apparent molecular weight. Both the full-length and intermediate bands showed a complete shift with PNGase F digestion (P) and a partial shift with Endo H digestion (E). The mature 45 kDa band showed this same set of shifts, but was more clearly visible as a doublet following PNGase F digestion, in concordance with published literature (Froissart *et al., ibid*).

HepG2-IDUA clones were similarly used to analyze the glycosylation pattern of the IDUA produced. Figure 15D shows that the PNGase F and EndoH produce similar patterns in the HepG2-IDUA cellular supernatant as the commercially available IDUA enzymes Aldurazyme® and R&D Systems rIDUA.

Combined, these data demonstrate that hIDS and IDUA proteins produced and secreted from the albumin locus contained a mixture of high mannose/hybrid glycosylations (Endo-H sensitive) and complex glycosylations (Endo-H insensitive/PNGase F-sensitive).

### F. Competition of cellular IDS uptake by mannose-6 phosphate

Conditioned media was made by culturing control HepG2/C3A (ATCC, CRL-10741; 'HepG2') cells or HepG2-IDS clones #8 or #15 in Eagle's Minimal Essential Media (MEM) (Corning) supplemented with 10% FBS and IX Penicillin-Streptomycin-Glutamine (Thermo Fisher Scientific). Cells were cultured for 72 hr to allow IDS to accumulate in the cell culture supernatant, and then the supernatant was collected, 0.2 µm filtered, and frozen at -80°C until use.

100,000 unmodified HepG2 or K562 cells were seeded per well in a 24 well plate and grown for 24 hr in a 37°C, 5% CO2 incubator. Media was then changed to HepG2 or HepG2-IDS conditioned media (generated as described above) in the presence or absence of 5 mM mannose-6-phosphate (M6P; Sigma-Aldrich). Cells were incubated in conditioned media ± 5 mM M6P for 6 or 24 hr. Cells incubated in conditioned media for 6 hr were switched back to normal cell growth media for 18 hr, and cell pellets from all conditions were collected at the end of this 24 hr period. Following removal of cell culture supernatant, cell pellets were washed once with 1 mL of PBS to remove any residual conditioned media prior to assaying for IDS activity. Cell pellets were lysed by sonication in 150 µL TBS + 0.1% Triton X-100 (Sigma-Aldrich) and 1x Halt protease inhibitor (Thermo Fisher Scientific) and assayed for IDS activity.

The results (Figure 16A and 16B) demonstrate that in the presence of the 5mM M6P, IDS uptake was reduced in both HepG2 and K562 cells.

### G. IDUA Produced from the Albumin locus in vitro was taken up by secondary cells in an mannose-6-phosphate dependent manner. Figure 16D is a schematic illustrating how treatment of the K562 cells with mannose-6-phosphate (M6P) prevents uptake of the IDUA enzyme.

Media was generated by culturing control HepG2/C3A (ATCC, CRL-10741; 'HepG2') cells or HepG2-IDUA clones #25 in Eagle's Minimal Essential Media (MEM) (Corning) supplemented with 10% FBS and IX Penicillin-Streptomycin-Glutamine (Thermo Fisher Scientific). Cells were cultured for 72 hr to allow IDUA to accumulate in the cell culture supernatant, and then the supernatant was collected, 0.2 µm filtered, and frozen at -80°C until use.

IDUA uptake was determined as follows. 100,000 unmodified K562 cells were seeded per well in a 24 well plate in the HepG2 conditioned media (generated as described above) in the presence or absence of 5 mM mannose-6-phosphate (M6P; Sigma-Aldrich). Cells were incubated in conditioned media ± 5 mM M6P for 24 hr. Following removal of cell culture supernatant, cell pellets were washed twice with 1 mL of PBS to remove any residual conditioned media prior to assaying for IDUA activity using the fluorescent substrate 4-Methylumbelliferyl α-L-iduronide (Santa Cruz Biotechnology).

As shown in Figure 16C, the produced from the albumin locus *in vitro* is taken up by secondary cells in a mannose-6-phosphate dependent manner.

### Example 5: Donor Uptake into Brain

Mass spectrometry of brain tissue homogenates for levels of dermatan and heparan sulfate, which are the glycosaminoglycans that are the substrates for the IDUA and IDS enzymes, was performed at Pacific BioLabs (Hercules, CA). Briefly, tissues of MPSI mice as described in Example 2 (untreated and treated with ZFNs and IDUA donors) were homogenized in 200 mM ammonium acetate, 20 mM calcium acetate, 4 mM DTT, pH 7.0. Protein concentrations were determined by BCA Protein Assay Kit (Thermo Fisher Scientific) and samples were normalized to 1 mg/mL. Heparan sulfate samples were digested with heparinase I and III, yielding two major disaccharides (Δ-UA-GlcNAc [IV-A] and Δ-UA-GlcNS [IV-S]). Dermatan sulfate samples were digested with chondroitinase B, yielding 3 major disaccharides (ΔUA-GalNAc (4S) [di-4S], ΔUA-GalNAc (6S) [Δdi-6S] and ΔUA-GalNAc (4S, 6S) [Δdi-4S,6S]). After digestion, an internal standard was spiked in, large molecules were removed by centrifuging through a 10,000 NMWL spin filter or filter plate, and samples were subjected to reversed-phase HPLC. Analytes and internal standards were detected via ESI-MS/MS using a specific multiple-reaction-monitoring method in negative mode. The peak areas of each analyte and IS were integrated in their respective MS-chromatogram and the ratios of peak areas were used for quantitation. Concentrations of respective disaccharides in unknown samples were interpolated from a calibration curve of standards of known concentration and are reported in µg/mL, with a lower limit of quantitation (LLOQ) of 0.005 µg/mL for each disaccharide. Disaccharide concentrations were summed for each sample and plotted relative to homogenate input.

As shown in Figure 17A, when brain tissue homogenates from MPS I mice were measured specifically for dermatan and heparan sulfate levels by mass spectrometry, there was a significant reduction of dermatan sulfate levels in male ZFN+Donor treated mice as compared to their gender-matched controls at 4 months post-injection. Female treated mice also showed a trend toward decreased dermatan sulfate levels. Likewise, ZFN+Donor treated MPS II mice also showed a trend of decreased brain dermatan sulfate levels which reached statistical significance at the highest ZFN+Donor dose (Figure 17B). However, no decreases were observed for heparan sulfate in either the MPS I or MPS II mouse models.

## Claims

1. A promoterless donor vector and an expression vector for use in a method of reducing or preventing central nervous system (CNS) impairment in a subject with mucopolysaccharidosis type II (MPS II) or mucopolysaccharidosis type I (MPS I), wherein the promoterless donor vector comprises a transgene encoding human iduronate-2-sulfatase (hIDS) or human α-L-iduronidase (hIDUA) and the expression vector encodes a pair of zinc finger nucleases (ZFNs) targeted to an endogenous albumin gene, the method comprising administering to the subject the promoterless donor vector and expression vector,
wherein the transgene is integrated into the albumin gene in a liver cell following cleavage of the albumin gene such that the encoded hIDUA or hIDS is expressed and secreted from the liver and further wherein the hIDUA or hIDS secreted from the liver is found in the central nervous system (CNS) of the subject such that the CNS impairment is reduced or prevented.

2. The promoterless donor vector and expression vector for use according to claim 1, wherein the CNS impairment comprises cognitive deficits.

3. The promoterless donor vector and expression vector for use according to claim 1 or claim 2, wherein the method further comprises administering an immunosuppressant to the subject prior to and after administration of the donor vector and the expression vector.

4. The promoterless donor vector and expression vector for use according to any of claims 1 to 3, wherein expression of the transgene modulates levels of glycosoaminoglycans in the brain of the subject.

5. The promoterless donor vector and expression vector for use according to any of claims 1 to 4, wherein neuronal or glial vacuolation in the subject is reduced or eliminated.

6. The promoterless donor vector and expression vector for use according to claim 5, wherein neuronal or glial vacuolation in the subject is reduced or eliminated cellular activity in the spinal cord of the subject.

7. The promoterless donor vector and expression vector for use according to any of claims 1 to 6, wherein loss of learning ability is decreased as compared to an untreated individual.

8. The promoterless donor vector and expression vector for use according to any of claims 1 to 7, wherein the pair of ZFNs comprises the zinc finger proteins shown in Table 1.

9. The promoterless donor vector and expression vector for use according to any of claims 1 to 8, wherein the ZFN expression vector and the donor vector comprise adeno-associated vectors (AAV).

10. The promoterless donor vector and expression vector for use according to claim 9, wherein the ZFN expression vector and the donor vector AAV are administered via intravenous injection.

11. The promoterless donor vector and expression vector for use according to any of claims 1 to 10, wherein the ZFN:ZFN:Donor ratio administered to the subject is 1:1:8.

## Patentansprüche

1. Promotorloser Donorvektor und Expressionsvektor zur Verwendung beim Verringern oder Verhindern einer Beeinträchtigung des Zentralnervensystems (ZNS) bei einem Individuum mit Mukopolysaccharidose Typ II (MPS II) oder Mukopolysaccharidose Typ I (MPS I), wobei der promotorlose Donorvektor ein Transgen umfasst, das menschliche Iduronat-2-Sulfatase (hIDS) oder menschliche α-L-Iduronidase (hIDUA) codiert, und der Expressionsvektor ein Paar Zinkfinger-Nukleasen (ZFN) codiert, die auf ein endogenes Albumin-Gen gerichtet sind, wobei das Verfahren Verabreichen des promotorlosen Donorvektors und Expressionsvektors umfasst,
wobei das Transgen in das Albumin-Gen in einer Leberzelle nach Spaltung des Albumin-Gens so integriert wird, dass die codierte hIDUA oder hIDS exprimiert und aus der Leber sezerniert wird, und ferner wobei sich die aus der Leber sezernierte hIDUA oder hIDS im Zentralnervensystem (ZNS) des Individuums findet, so dass die ZNS-Beeinträchtigung verringert oder verhindert wird.

2. Promotorloser Donorvektor und Expressionsvektor zur Verwendung nach Anspruch 1, wobei die ZNS-Beeinträchtigung kognitive Defizite umfasst.

3. Promotorloser Donorvektor und Expressionsvektor zur Verwendung nach Anspruch 1 oder Anspruch 2, wobei das Verfahren ferner Verabreichen eines Immunsuppressivums an das Individuum vor oder nach Verabreichung des Donorvektors und des Expressionsvektors umfasst.

4. Promotorloser Donorvektor und Expressionsvektor zur Verwendung nach einem der Ansprüche 1 bis 3, wobei durch Expression des Transgens Glykosoaminoglykanspiegel im Gehirn des Individuums moduliert werden.

5. Promotorloser Donorvektor und Expressionsvektor zur Verwendung nach einem der Ansprüche 1 bis 4, wobei Neuronen- oder Gliavakuolisierung beim Individuum vermindert oder beseitigt ist.

6. Promotorloser Donorvektor und Expressionsvektor zur Verwendung nach Anspruch 5, wobei Neuronen- oder Gliavakuolisierung beim Individuum verminderte oder beseitigte Zellaktivität im Rückenmark des Individuums bedeutet.

7. Promotorloser Donorvektor und Expressionsvektor zur Verwendung nach einem der Ansprüche 1 bis 6, wobei der Verlust von Lernfähigkeit im Vergleich zu einem unbehandelten Individuum geringer ist.

8. Promotorloser Donorvektor und Expressionsvektor zur Verwendung nach einem der Ansprüche 1 bis 7, wobei das ZFN-Paar die in Tabelle 1 dargestellten Zinkfingerproteine umfasst.

9. Promotorloser Donorvektor und Expressionsvektor zur Verwendung nach einem der Ansprüche 1 bis 8, wobei der ZFN-Expressionsvektor und der Donorvektor adeno-assoziierte Vektoren (AAV) umfassen.

10. Promotorloser Donorvektor und Expressionsvektor zur Verwendung nach Anspruch 9, wobei der ZFN-Expressionsvektor- und der Donorvektor-AAV über intravenöse Injektion verabreicht werden.

11. Promotorloser Donorvektor und Expressionsvektor zur Verwendung nach einem der Ansprüche 1 bis 10, wobei das dem Individuum verabreichte ZFN:ZFN:Donor-Verhältnis 1:1:8 beträgt.

## Revendications

1. Vecteur donneur sans promoteur et vecteur d'expression pour une utilisation dans un procédé de réduction ou de prévention d'une détérioration du système nerveux central (SNC) chez un sujet présentant une mucopolysaccharidose type II (MPS II) ou une mucopolysaccharidose type I (MPS I), le vecteur donneur sans promoteur comprenant un transgène codant pour l'iduronate-2-sulfatase humaine (hIDS) ou l'α-L-iduronidase humaine (hIDUA), et le vecteur d'expression codant pour une paire de nucléases à doigts de zinc (ZFN) ciblées vers un gène de l'albumine endogène, le procédé comprenant l'administration au sujet du vecteur donneur et du vecteur d'expression sans promoteur,
dans lesquels le transgène est intégré dans le gène de l'albumine dans une cellule hépatique après clivage du gène de l'albumine de sorte que la hIDUA ou la hIDS codée soit exprimée et secrétée du foie et en outre dans lesquels la hIDUA ou la hIDS secrétée du foie soit trouvée dans le système nerveux central (SNC) du sujet de façon que la détérioration du SNC soit réduite ou empêchée.

2. Vecteur donneur et vecteur d'expression sans promoteur pour une utilisation selon la revendication 1, dans lesquels la détérioration du SNC comprend des déficits cognitifs.

3. Vecteur donneur et vecteur d'expression sans promoteur pour une utilisation selon la revendication 1 ou la revendication 2, le procédé comprenant en outre l'administration d'un immunosuppresseur au sujet avant et après administration du vecteur donneur et du vecteur d'expression.

4. Vecteur donneur et vecteur d'expression sans promoteur pour une utilisation selon l'une quelconque des revendications 1 à 3, dans lesquels l'expression du transgène module les niveaux de glycosoaminoglycanes dans le cerveau du sujet.

5. Vecteur donneur et vecteur d'expression sans promoteur pour une utilisation selon l'une quelconque des revendications 1 à 4, dans lesquels la vacuolisation neuronale ou gliale chez le sujet est réduite ou éliminée.

6. Vecteur donneur et vecteur d'expression sans promoteur pour une utilisation selon la revendication 5, dans lesquels la vacuolisation neuronale ou gliale chez le sujet est une activité cellulaire réduite ou éliminée dans la moelle épinière du sujet.

7. Vecteur donneur et vecteur d'expression sans promoteur pour une utilisation selon l'une quelconque des revendications 1 à 6, dans lesquels la perte de capacité d'apprentissage est réduite par comparaison avec un individu non traité.

8. Vecteur donneur et vecteur d'expression sans promoteur pour une utilisation selon l'une quelconque des revendications 1 à 7, dans lesquels la paire de ZFN comprend les protéines à doigts de zinc présentées dans le Tableau 1.

9. Vecteur donneur et vecteur d'expression sans promoteur pour une utilisation selon l'une quelconque des revendications 1 à 8, dans lesquels le vecteur d'expression des ZFN et le vecteur donneur comprennent des vecteurs adéno-associés (AAV).

10. Vecteur donneur et vecteur d'expression sans promoteur pour une utilisation selon la revendication 9, dans lesquels le vecteur d'expression des ZFN et le vecteur donneur AAV sont administrés par injection intraveineuse.

11. Vecteur donneur et vecteur d'expression sans promoteur pour une utilisation selon l'une quelconque des revendications 1 à 10, dans lesquels la proportion ZFN:ZFN:donneur administrée au sujet est de 1:1:8.
